# EUROPEAN PATENT SPECIFICATION

(11) **EP 2 475 417 B1**
(45) Date of publication and mention of the grant of the patent: **10.10.2018**
(21) Application number: 10816200.9
(22) Date of filing: 10.09.2010
(51) Int. Cl.: A61M 25/088, A61M 25/06, A61M 25/10, A61M 29/02

(54) **EXPANDABLE CEREBROVASCULAR SHEATH**
DEHNBARE ZEREBROVASKULÄRE SCHLEUSE
GAINE CÉRÉBROVASCULAIRE EXTENSIBLE

(30) Priority: 11.09.2009 US 241740 P
(43) Date of publication of application: 18.07.2012
(73) Proprietor: Onset Medical Corporation, Irvine, CA 92618 (US)
(72) Inventor: LENKER, Jay, Laguna Beach CA 92651 (US); BISHOP, Joseph, Manifee CA 92584 (US); JONES, Mark, T., Garden Grove CA 92844 (US); NGUYEN, Huan, T., Santa Ana CA 92704 (US)
(74) Representative: Morris, Jonathan Paul
(86) International application number: PCT/US2010/048524
(87) International publication number: WO 2011/032038

(56) References cited:
- WO-A2-2006/031619
- WO-A2-2008/073852
- WO-A2-2010/075565
- US-A1- 2004 019 322
- US-A1- 2006 135 981
- US-A1- 2008 243 081
- US-A1- 2009 287 182
- US-B1- 6 436 087
- US-B2- 6 929 634

## Description

### Background of the Invention

### Field of the Invention

The present invention relates to medical devices for percutaneously accessing the cardiovascular system and, more specifically, accessing the cerebrovasculature.

### Description of the Related Art

A wide variety of diagnostic or therapeutic procedures involve the introduction of a device into the vasculature through a percutaneous or open surgical incision at an access site and then routing the device to a remote location within the body for therapy or diagnosis at the remote location. Such remote locations within the body include the neurovasculature or cerebrovasculature. Catheters are routed to the cerebrovasculature over guidewires, through guide catheters, or both. A percutaneous technique commonly known for such vascular access is the Seldinger technique. The Seldinger technique involves using a hollow needle to puncture the skin and gain access to the selected artery. A guidewire is next placed through the hollow needle into the selected region of vasculature. The guidewire may be advanced to a target location in the cerebrovasculature, through either the vertebral or carotid arteries. The needle is removed and a tapered dilator with a guide catheter and a central lumen within the dilator is advanced over the guidewire into the cerebrovasculature. The dilator is next removed, as is the guidewire. The guide catheter can be advanced all the way, or part way, to the target site. The guide catheter, following, or without, removal of the guidewire can be used for directing therapeutic or diagnostic catheters to regions of the cerebrovasculature. These procedures are especially suited for cerebrovascular aneurysm repair, thrombectomy, embolectomy, neck bridge placement, stent placement, cerebrovascular embolic coil placement, foreign body removal, diagnostic cerebrovascular catheterization, and the like.

Following the therapeutic or diagnostic procedure, the sheath is next removed and hemostasis is established using standard techniques for a vessel puncture wound.

Adequate advancement of guide catheters is generally restricted by the tortuous anatomy of the carotid or vertebral arteries, as well as their small size. Negotiation of the carotid siphon or basilar artery into the circle of Willis is generally restricted to small diameter, highly flexible, microcatheters. Guide catheters are generally unable to negotiate into, or far into, the circle of Willis due to the tortuous anatomy leading thereto.

It is desirable to maximize advancement of guide catheters within the neurovasculature so that the vascular walls are shielded from catheters being placed therein. A need remains, therefore, for improved access technology, which allows a guide catheter or sheath to be percutaneously or surgically introduced into the neurovasculature, further into the circle of Willis, or beyond, than is now possible using standard guide catheter technology.

Examples of expandable sheaths that are arranged for introduction into the body are disclosed in US2006/0135981, WO 2008/073852 and US2009/287182.

### Summary of the Inventions

According to one aspect of the invention there is provided a guide catheter according to claim 1.

The guide catheter includes an expandable sheath. The expandable sheath is used to assist with cerebrovascular access procedures in that it allows for a small diameter, highly flexible, access to the cerebrovasculature in its first, smaller cross-sectional state. Following advancement to a target location within the neurovasculature or cerebrovasculature, the collapsible region of the sheath can be diametrically expanded such that it retains a substantially uniform cross-sectional lumen from its proximal end to its distal end. In its enlarged state, with the collapsible distal region having been fully dilated, the guide catheter can serve as a pathway large enough in size for introduction of large interventional, therapeutic, or diagnostic devices therethrough. Interventional neuroradiologists (INR) generally prefer to perform interventional procedures where the access is percutaneous and does not require a surgical cutdown. The expandable cerebrovascular can reduce procedure time, decrease procedure cost, reduce trauma to the patient, and improve patient outcomes by advancing further into the cerebrovasculature toward a target lesion than would have been otherwise possible with a non-expandable sheath or guide catheter. This extra advancement reduces the risk of distal embolization or vessel wall damage and increases the ability to route instrumentation to the target region.

In some arrangements, the distal section can comprise a polymeric wall with reinforcing elements that provide a degree of retention of cross-sectional shape. The distal section can comprise reinforcing elements that provide substantial control over the shape of the polymeric wall but are easily deformed into a collapsed configuration upon exposure to external forces such as those imposed by a blood vessel wall. The distal section can comprise polymeric materials that can be plastically deformed and do not substantially spring back following dilation. In these embodiments, the distal end is subject to remodeling by inflation of the expansion balloon under pressures ranging between 5 and 40 atmospheres.

### (5,066 - 40,53 bar)

In other arrangements, the distal end of the sheath can comprise a flared component that becomes larger in diameter moving distally. The flared component can comprise a taper, or it can comprise a taper and a region of relatively constant diameter affixed or integral to the tapered region at its most distal end. The flared component can be integral to the distal end of the expandable portion of the sheath, or it can be affixed thereto. The flared component can be expanded using a balloon dilator, it can be expanded using self-expansion modalities, or it can comprise self-expansion with balloon dilator assist. The self-expansion can be due to resilient spring forces, or due to shape memory forces generated by sheath reinforcement components fabricated from nitinol, or other shape memory materials. The flared configuration can facilitate re-capture or removal of instruments, embolic material, debris, or implantable devices such as percutaneously delivered aortic heart valves. The expandable, flared region of the sheath can range in length between 1-cm and 10-cm, with a preferred range of 2-cm to 5-cm. In an embodiment, the flared region can use the same balloon as the rest of the distal expandable region for expansion, or it can be expanded by a separate balloon.

In some arrangements, the hub at the proximal end of the sheath incorporates one or more hemostasis-type valves. The hub can comprise a single catheter insertion port or it can comprise a plurality of catheter insertion ports. Each catheter insertion port preferably comprises hemostasis valves, stopcocks, or the like to prevent blood leakage from the catheter. The hub can further comprise one or more purge ports, which operably connect to the internal lumen of the hub and are terminated by stopcocks or other valve.

In some arrangements, the diametrically or radially expandable elements of the catheter are configured as a tube having a plurality of longitudinal folds. The expandable regions or elements, located in the proximal section, distal section, or the center section of the sheath or catheter, are creased into these folds and bent to form a first, smaller, folded cross sectional area. The expandable regions or elements are folded over a central dilator catheter comprising, for example, an angioplasty-type balloon, a catheter shaft, a balloon inflation port at the proximal end, a guidewire lumen, and the like. Upon selective inflation of the angioplasty-type, non-elastomeric, non-distensible, balloon by application of fluid pressure into an appropriate port on the proximal end of the dilator catheter, the expandable regions unfolds into a second, larger, cross-sectional shape. The central dilator catheter can be deflated and removed from the sheath to create a large cross-section center lumen suitable for the introduction of catheters, delivery catheters, implantable devices, and the like.

In an embodiment, the expandable introducer sheath can comprise a proximal, expandable section. The proximal expandable section comprises a composite tubular structure fabricated from an inner polymeric layer of polyethylene, an outer polymeric layer of polyethylene, and a reinforcement layer sandwiched between the two polymer layers. The reinforcement layer can comprise a coil of flat, fully annealed, stainless steel wire with a width of about 0.25 mm (0.010 inches), with a range of 0.1 mm to 0.6 mm (0.005 to 0.025 inches), and a thickness of about 0.08 mm (0.003 inches), with a range of 0.05 mm to 0.1 mm (0.002 to 0.004 inches). The proximal, expandable region is affixed at its proximal end to a non-expandable length of sheath tubing of the same or similar inside diameter, or it is affixed directly to the sheath hub. The distal end of the proximal expandable region is affixed to a central expandable region that comprises inelastic polymeric materials. The central expandable region can comprise a membrane of polymers bonded, welded, or surrounding a braid, or other fabric reinforcing structure that provides a level of column strength and a level of tensile strength for the central expandable region. The distal end of the central expandable region is affixed to a distal expandable region configured similarly to the proximal expandable region except that the distal expandable region is somewhat weaker so that it is easily collapsed, following expansion. Transition zones, capable of joining expandable regions to non-expandable regions, or capable of joining two regions of different expandability, structurally and functionally affix one sheath tubular region to the next. The transition zones can comprise plastic welds or welds of the reinforcing layers to one another. The transition zones can comprise mechanical interconnections between reinforcing layers.

In another embodiment, the sheath tubing can comprise a proximal region wherein a reinforcing layer of spring stainless steel ribbon is wound into a coil with a width of about 0.1 mm to 0.6 mm (0.004 to 0.025 inches) and a thickness of about 0.01 mm to 0.1 mm (0.0005 to 0.004 inches). The coil spacing can range between 0.03 mm and 1.3 mm (0.001 inches and 0.050 inches). In other embodiments, the sheath tubing can comprise a proximal region wherein a reinforcing layer of metallic or polymeric braid is disposed or embedded within layers of the polymeric sheath. The braid can comprise, for example materials such as, but not limited to, PEN, polyester, polyimide, polyamide, stainless steel, nitinol, tantalum, noble metal, or the like. The braid materials are preferably elastomeric or have spring properties.

In another embodiment, the sheath can comprise a proximal non-expandable region and a distal expandable region. The distal expandable region can comprise between about 5% and 60% of the catheter shaft length.

The distal, expandable region can comprise a reinforcing layer of malleable stainless steel ribbon or flat wire wound into a coil with similar dimensions as in the proximal region. In an alternative embodiment, the entire length, or a substantial portion thereof, can comprise an additional reinforcing layer, or layers, of braided material fabricated from materials such as, but not limited to, PEN, polyester, stainless steel, titanium, nitinol, cobalt nickel alloy, polyamide, polyimide, or the like. In one arrangement, the reinforcing structure, generally sandwiched between an outer and an inner layer of polymeric wall, can comprise an inner layer of polymer overlaid by a first reinforcing braid layer, overlaid by a coil reinforcement, finally overlaid with an outside layer of polymeric material. In another embodiment, the inner layer of polymeric material is overlaid by the coil reinforcement, which is overlaid by the braided reinforcement, which is finally overlaid with the outside layer of polymeric material. In yet another embodiment, the inner layer of polymeric material is overlaid by the braided layer, which is overlaid by the coil winding, which is overlaid by another layer of braid, which is finally overlaid by the outer polymeric layer. The polymeric layers can comprise materials such as, but not limited to, fluoropolymer (e.g. PTFE, FEP, PFA), Hytrel, Pebax, Nylon, polyester, and the like.

In one embodiment, the sheath dilator is configured with a PET balloon affixed to a Hytrel shaft. The Hytrel shaft can comprise an inner and an outer tube concentrically disposed with an annulus between the two tubes. The distal end of the dilator balloon can be affixed to the inner Hytrel tubing. The proximal end of the dilator balloon is larger in diameter and is affixed to the outer Hytrel tubing in this embodiment. The outer Hytrel tubing extends just inside the center volume of the dilator balloon and the annulus between the outer tube and the inner tube is in fluid communication, operably connected to, the center volume of the dilator balloon. The annulus is operably in fluid communication with an inflation port integral to, or affixed to, the dilator hub. In another embodiment, an outer polymer tube, such as the outer Hytrel tube of the preceding embodiment, can be omitted and the dilator balloon can comprise a proximal tail that extends proximally to bond and seal within the dilator hub or sidearm. In this embodiment, the pressurization annulus for the balloon resides between the dilator balloon and the inner polymer tube, the pressurization annulus being operably connected to an inflation port on the dilator hub. The interior of the inner dilator tube comprises a guidewire lumen suitable for advancing the entire system over a guidewire. Such guidewires typically are 0.25 mm to 1 mm (0.010 to 0.038 inches) in diameter with an exemplary diameter of about 0.4 mm (0.014 inches). For a .014 guidewire, for example, an inner dilator tube inside diameter can range from about 0.4 mm to 0.5 mm (0.016 to about .020 inches).

The sheath is folded into one or more longitudinally oriented folds and wrapped around the dilator, with collapsed dilator balloon. The malleable elements in the proximal and distal expandable regions maintain the configuration of the system in its collapsed state. An outer jacket, which can have attached, peel-away, tear-away, or removable before use configurations, can be used to encase part or all of the diametrically collapsed sheath tubing. In other embodiments, the sheath can further comprise a thin PET, FEP, PFA, or PTFE tube over the outside of the sheath. This fluoropolymer outer covering need not be removed, its function being to protect a soft polyethylene sheath material from hard vascular deposits such as atheroma.

Once the expandable guide catheter has been advanced so that its distal end reaches to the target area within the vasculature, the dilator is expanded at pressures of between about 5 and 40 atmospheres, (5,066 - 40,53 bar) and preferably between 10 and 30 atmospheres. (10,132 - 30,4 bar) The dilator is next deflated and removed from the central lumen of the guide catheter leaving the large central lumen open for advancement of therapeutic or diagnostic catheters therethrough.

The reinforcement of the expandable regions can comprise wire, preferably malleable wire. The wire can have a round cross-section, a rectangular cross-section, a ribbon-like cross-section, or the like. The malleable wire can be bent by a dilator balloon, tapered dilator, hollow dilator, or the like, into the second, larger cross-section and the strength of the malleable wire can substantially overcome any resilient spring-back imparted by the polymeric component of the sheath wall.

In other embodiments, the wire can have elastomeric properties or shape memory properties. These embodiments can utilize shape-memory wire, pseudoelastic wire, superelastic wire, elastomeric wire, or the like. The wire can be nitinol, stainless steel, cobalt nickel alloy, or the like. The wire, in its shape-memory configuration can have an austenite finish temperature (Af) of around 25 to 35 degrees centigrade, preferably between 28 and 32 degrees centigrade so that body temperature blood causes the wire mesh to be biased to its larger, expanded configuration.

In another embodiment, the expandable region can comprise polymeric encapsulation of a braided, coiled, or otherwise expandable shape memory reinforcing structure. The reinforcing elements or structure can have shape-memory characteristics. The sheath is inserted into the patient in its first, small cross-sectional area. The reinforcing elements are maintained below the martensite start temperature so that the reinforcing elements are substantially malleable, even at body temperature (approximately 37°C). The sheath wall is next dilated with the balloon dilator as described herein. The dilator is next removed and the sheath becomes host to therapeutic or diagnostic catheters, which are inserted therethrough. Following removal of the catheters, electricity (AC or DC) can be applied to lead wires at proximal end of the sheath. The lead wires run substantially the length of the catheter from the proximal end to the distal end, where they are operably connected to heaters in the vicinity of the reinforcing elements, or the electrical leads are operably connected to each end of the reinforcing elements. The electricity causes Ohmic or resistive heating of the reinforcing elements to above their austenite finish temperature. The reinforcing structure, having been shape-set in its small diameter configuration, returns to that small diameter configuration, bringing the entire expandable sheath wall down with it, to facilitate removal of the sheath from the patient. An austenite finish temperature of around 42°C can be used in this application. The reinforced expandable region comprises a folded, or furled, structure that is unfolded, or unfurled to cause expansion.

The dilator catheter can comprise an inner and outer member. The materials of the inner member and the outer member can comprise Hytrel, PEEK, composite, reinforced construction, polyester, polyurethane, polyethylene, or the like. The catheter hub can be fabricated from materials such as, but not limited to, polycarbonate, acrylonitrile butadiene styrene (ABS), polyurethane, polyvinyl chloride, and the like. The dilator balloon can be fabricated from stretch blow-molded polyester polyamide, polyamide, or polyester blends, using materials such as, for example, Eastman PET 9921 or similar.

In another embodiment, a coating can be applied to the expandable areas to generate an inwardly biased, radially oriented contraction force. The expandable area can be forced to expand radially against the bias force of the coating. Once the radial expansion force is removed, the expandable area remains biased radially inward toward its smallest diameter, to which it will travel unless prevented from doing so.

The system can comprise radiopacity enhancements to improve visualization under fluoroscopy. Radiopaque markers can be affixed to the distal end of the sheath to denote its distal end, the extents of the expandable region or regions, or even the orientation of the sheath. The radiopaque markers can comprise bands, braids, or windings of metal such as, but not limited to, tantalum, platinum, platinum iridium, gold, and the like.

In certain embodiments of the sheath wall construction, an inner layer of polymer and an outer layer of polymer sandwich a reinforcing layer. The reinforcing layer can be a coil of metal such as, but not limited to, titanium, stainless steel, cobalt nickel alloy, nitinol, tantalum, and the like. The coil is preferably malleable, with little or no spring properties, and does not exhibit any elastomeric tendencies. The coil can be fabricated from flat wire with a thickness of 0.01 mm to 0.25 mm (0.0005 to 0.010 inches) and preferably 0.02 mm to 0.1 mm (0.0007 to 0.005 inches). The width of the flat wire can range from about 0.08 mm to 1 mm (0.003 to 0.050 inches) and preferably from about 0.1 mm to 0.3 mm (0.005 to 0.010 inches). The spacing between the coils can, for example range from substantially 0 to approximately 5 times the width of the coil wire, with an exemplary spacing equal to about the width of the coil wire. The coils can be fabricated from round stock, flat stock, or the like. The reinforcement can be sandwiched between the inner layer and the outer layer of polymeric material, wherein the inner and outer layers can be bonded or welded to each other through the space between the coils. The inner and outer polymeric layers can be fabricated from the same or different materials. Suitable materials for the inner and outer layers include, but are not limited to, polyurethane, silicone, Hytrel, PEEK, polyethylene, HDPE, LDPE, polyester (e.g. PET), polyethylene blends, and the like. In yet another embodiment, a plastically deformable, malleable, or annealed, braid structure can also be used for reinforcement to beneficially eliminate the need for the malleable coil and permit a reduction in wall thickness while retaining the tensile strength and torqueability of the braid. In yet other embodiments, the reinforcement can comprise a stent-like structure.

In certain embodiments, the guide catheter sheath shaft can comprise multiple regions of varying flexibility along the axial length of the shaft. In some embodiments, the guide catheter dilator shaft can have at least two regions of different flexibility. In other embodiments, the guide catheter shaft can comprise three or more (with a practical upper limit of six) regions of different flexibility. In yet other embodiments, the sheath shaft flexibility can be reduced toward the proximal end of the guide catheter and increased moving toward the distal end of the guide catheter. Moving from the proximal to the distal end of the guide catheter shaft, the flexibility of a given discreet section can be greater than the flexibility of the region just proximal and adjacent to said discreet section. A guide catheter sheath having a substantially collapsed, small diameter distal region can exhibit significantly increased flexibility in that area over its flexibility in non-expandable, or fully expanded, expandable regions. Such flexibility is especially useful when traversing tortuous or curved anatomy such as the aortic arch into the brachiocephalic trunk (innominate arteries). Following such traverse, the guide catheter sheath can be expanded to create a stiffer, larger diameter structure.

The expandable guide catheter is configured to be inserted into the femoral or iliac arteries and be routed through the aorta so that its distal end is within the carotid, or vertebral, arteries. In these embodiments, the working length of the introducer sheath can be such that the sheath reaches just proximal to the carotid siphon. In other embodiments, the working length of the sheath can be such that the sheath reaches well into the circle of Willis, or beyond to the region of anterior communicating artery, or the middle cerebral artery. In these embodiments, the working length of the introducer sheath can range between about 90 and 150 cm with a preferred length of about 100 to 130 cm. The expandable distal region can comprise between about 5 to 40 cm of the sheath tubing, and preferably between about 10 to 30 cm, leaving the proximal portion of the sheath tube as a non-deformable, non-collapsible, or non-deflectable region at the proximal end to facilitate attachment to the sheath hub and to provide for column strength and torqueability. In one preferred example, the overall working length is about 120 cm and the expandable distal region is about 20 cm long. In another preferred example, the overall working length is about 120 cm and the expandable distal region is about 12 to 15 cm long. In yet another preferred example, the overall working length is about 210 cm and the expandable distal region is about 30 cm long. The system can be fabricated with an outside diameter of about 2 mm (6 French) or it can be fabricated with an outside diameter of about 2.3 mm (7 French). The diameter tolerance can typically be about plus or minus 0.2 mm (0.5 French).

Another example comprises a method of use in which an expandable cerebrovascular sheath or guide catheter is provided in an aseptic, or sterile, package and is sterilized by ethylene oxide, gamma irradiation, electron beam irradiation, or the like. The patient is prepared in the standard hospital fashion for surgery and is appropriately draped. A percutaneous needlestick is made into an iliac or femoral artery using the Seldinger technique described earlier in this document. A guidewire is advanced through the hollow 18-gauge needle and the needle is removed. The percutaneous access site can optionally be dilated with an Amplatz dilator or similar device at this time. The introducer sheath, in its first, small cross-sectional configuration, with its dilator is advanced over the guidewire and into the artery where it is advanced through the aorta. The distal end of the introducer sheath is advanced into a carotid or vertebral artery. In some examples the introducer sheath is advanced, in its collapsed configuration, through the carotid siphon and into the circle of Willis or beyond. The introducer sheath is next dilated to its second, larger cross-sectional configuration, using the pre-inserted dilator or by other suitable means. The dilator is next removed and any hemostasis valves are checked for closure at the proximal end of the sheath. Interventional therapeutic catheters are next advanced through the expandable introducer sheath and toward their anatomical target. Following completion of the procedure, the interventional, or diagnostic, catheters are removed from the expandable iliac introducer sheath, again checking to ensure that there is no hemorrhage from the valves or ports at the proximal end of the sheath. The sheath is removed from the patient in one of two ways. In some examples the sheath is withdrawn from the patient without actively collapsing the sheath but the sheath collapses slightly following removal of the interventional catheters to ease withdrawal. In other examples the sheath is actively reduced in diameter or cross-section and is then withdrawn from the patient. Hemostasis is maintained using standard hospital technique or by the application of a commercial percutaneous access hemorrhage control device.

Various embodiments examples of the sheath can cause sheath re-collapse, in the radial, diametric, or cross-sectional directions. In some examples shape-memory nitinol can be heated to above body temperature to cause restoration to austenite finish temperature and return to a pre-set, collapsed shape. In embodiments, the outer layer of the sheath is separate from inner layers. The outer layer of the sheath comprises substantially non-compliant material. In another example it can comprise substantially semi-compliant materials, or a combination thereof. An inflation port at the proximal sheath hub can be operably connected to the potential space between the outer layer of the sheath and the inner layers. Pressurization of the potential space between the outer layer and the inner layers coerces, crushs, forces, or otherwise moves the inner layers inward to a greater degree. The region of potential space can be a single chamber or it can comprise a plurality of cambers with heat seals or other barriers therebetween. Following removal of the pressurization within the potential space, the collapsed sheath and its now flaccid outer layer can be removed from the patient. In some embodiments, the space between the now collapsed or refolded inner sheath and the flaccid outer layer can be evacuated of fluid to collapse the outer layer to the smallest possible diameter to facilitate removal of the sheath from the patient. In some embodiments, the outer layer can comprise two layers sealed to each other such that pressurization occurs between the double walled outer layer. These embodiments can be useful when it is difficult to seal the outer layer to the inner layers due to material incompatibilities. Suitable material for the sheath outer layer can include, but not be limited to, polyester (PET), polyimide, polyamide, or the like. Wall thicknesses for the outer layer can range from about 0.005 mm to 0.03 mm (0.0002 to 0.001 inches).

The main reasons for the malleable embodiments include control over cross-sectional shape, ability to embed the reinforcement in the polymer layers without needing to create some difficult-to-manufacture decoupling of the polymer and the reinforcement, the high strength of the sheath following placement, and prevention of lumen re-collapse caused by body tissue. The ability of this device to remodel to the desired shape to generate a superhighway for placement of implants and other medical devices is superior to anything available today. Furthermore, the device provides a relatively smooth interior lumen, which allows passage of instruments and implants of very large size without excessive binding or friction. No other sheath exists today that has these benefits. The malleable reinforcements embedded within the sheath are configured to generate sufficient force that they control and maintain the diameter of the radially collapsed, unexpanded sheath. The malleable reinforcements are further configured to maintain the sheath in its open, radially expanded configuration, following dilation with a balloon or other dilator, residing within the sheath lumen. The structure of the malleable metal reinforcement is sufficient to overcome, or dominate, any resilient or structural forces exerted by the polymeric components of the sheath tubing, which generally surround, or encase, the reinforcement. The structure of the malleable metal reinforcement also is sufficient to overcome any inwardly biased forces imposed by any tissue through which the sheath is inserted, such as, for example, muscle mass and fascia lying between the skin and the femoral or iliac arteries, or stenotic arterial buildup including thrombus or atherosclerotic plaque.

For purposes of summarizing the invention, certain aspects, advantages and novel features of the invention are described herein. It is to be understood that not necessarily all such advantages may be achieved in accordance with any particular embodiment of the invention. Thus, for example, those skilled in the art will recognize that the invention may be embodied or carried out in a manner that achieves one advantage or group of advantages as taught herein without necessarily achieving other advantages as may be taught or suggested herein. These and other objects and advantages of the present invention will be more apparent from the following description taken in conjunction with the accompanying drawings.

### Brief Description of the Drawings

A general architecture that implements the various features of the invention will now be described with reference to the drawings. The drawings and the associated descriptions are provided to illustrate embodiments of the invention and not to limit the scope of the invention. Throughout the drawings, reference numbers are re-used to indicate correspondence between referenced elements.
Fig. 1 is a front view schematic representation of the human central circulatory system;
Fig. 2 is a schematic representation of part of the human cerebrovasculature,
Fig. 3A illustrates an expandable introducer sheath system, in its radially expanded configuration with its dilator catheter in place, according to an embodiment of the invention;
Fig. 3B illustrates a balloon dilator for an expandable introducer sheath system, according to an embodiment of the invention;
Fig. 3C illustrates the expandable introducer sheath of Fig. 1A in its radially expanded configuration and with the dilator catheter removed, according to an embodiment of the invention;
Fig. 4A illustrates a the distal, expandable end of an expandable introducer sheath, in its first, unexpanded configuration with a dilator inserted therein, according to an embodiment of the invention;
Fig. 4B illustrates the distal end of the expandable introducer sheath of Fig. 4A in its expanded configuration with the dilator having been removed, according to an embodiment of the invention;
Fig. 4C illustrates the distal end of the balloon dilator with the balloon in its inflated configuration, according to an embodiment of the invention;
Fig. 5A illustrates the proximal end of the expandable sheath dilator in partial breakaway view, according to an embodiment of the invention;
Fig. 5B illustrates the proximal end of the expandable introducer sheath or guide catheter in partial breakaway view, according to an embodiment of the invention;
Fig. 6 illustrates an expandable guide catheter or introducer sheath and dilator, in its first, radially collapsed configuration being advanced across the carotid siphon, into the cerebrovasculature, according to an embodiment of the invention;
Fig. 7 illustrates the expandable introducer sheath or guide catheter within the middle cerebral artery of the cerebrovasculature, having been radially expanded and its dilator removed, according to an embodiment of the invention;
Fig. 8 illustrates the fully expanded introducer sheath or guide catheter with a therapeutic microcatheter having advanced through the sheath, according to an embodiment of the invention;
Fig. 9A illustrates a lateral cross-section of a distal region of an expandable arterial sheath comprising a single fold, according to an embodiment of the invention;
Fig. 9B illustrates a lateral cross-section of a distal region of an expandable arterial sheath comprising a double fold, according to an embodiment of the invention;
Fig. 10A illustrates the guide catheter or introducer sheath having an outer jacket, in its second, radially expanded configuration with the inflated dilator still in place, according to an embodiment of the invention;
Fig. 10B illustrates the guide catheter or introducer sheath comprising the outer jacket, in its second, radially expanded configuration with the dilator removed, according to an embodiment of the invention;
Fig. 10C illustrates the guide catheter or introducer sheath with the space between the outer jacket and the introducer sheath pressurized to collapse the introducer sheath distal tube to its third, radially collapsed configuration, according to an embodiment of the invention;
Fig. 10D illustrates the guide catheter or introducer distal end with the space between the outer jacket and the introducer sheath depressurized to collapse the outer jacket, according to an embodiment of the invention;
Fig. 11a illustrates a collapsing obturator for use with expandable introducer sheaths,
Fig. 11b illustrates the collapsing obturator having been inserted into a diametrically expanded introducer sheath, and then pressurized to expand two sealing balloons,
Fig. 11c illustrates the collapsing obturator within the introducer sheath with the two sealing balloons inflated and the region between the sealing balloons but outside the collapsing obturator depressurized to radially collapse the expandable introducer sheath tubing,
Fig. 12a illustrates a forming or re-folding obturator in side view configured to control the shape of the distal collapsible region of an introducer sheath,
Fig. 12b illustrates a cross-sectional view of a forming or re-folding obturator having a three-pronged profile, and
Fig. 12c illustrates a cross-sectional view of a forming or re-folding obturator having a splayed U configuration.

### Detailed Description of the Preferred Embodiments

As used herein, the terms proximal and distal refer to directions or positions along a longitudinal axis of a catheter or medical instrument. Proximal refers to the end of the catheter or medical instrument closest to the operator, while distal refers to the end of the catheter or medical instrument closest to the patient. For example, a first point is proximal to a second point if it is closer to the operator end of the catheter or medical instrument than the second point. However, the terms anatomically proximal and anatomically distal refer to orientations within the body. A point is more anatomically distal if it is further from the heart than a point described as anatomically proximal.

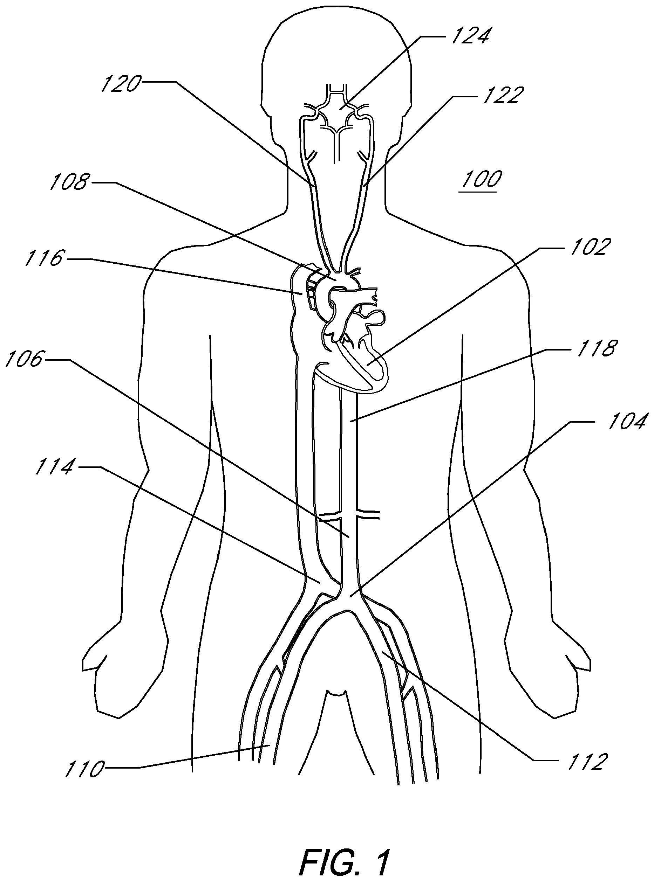

Figure 1 is a schematic frontal (anterior) illustration (looking posteriorly) of a human patient 100 that illustrates components of the central circulation. As shown, the central circulation generally comprises a heart 102, an aortic bifurcation 104, a descending aorta 106, an aortic arch 108, an inferior vena cava 114, a superior vena cava 116, an iliac artery 112, a femoral artery 110, a thoracic aorta 118, a plurality of carotid arteries 120, 122, and a main cerebrovasculature 124. In this illustration, the left anatomical side of the body of the patient 100 is toward the right of the illustration. Figure 1 primarily illustrates components of the central circulation.

Referring to Figure 1, the heart 102 is a pump, the outlet of which is the aorta, including the aortic arch 108, the thoracic aorta 118, the descending aorta 106, and the aortic bifurcation 104, which comprise the primary artery in the systemic circulation. The circulatory system, which is operably connected to the heart 102 further comprises the return, or venous, circulation. The venous circulation comprises the superior vena cava 116 and the inferior vena cava 114, which return blood from the upper extremities and lower extremities, respectively. The iliac arteries 112 are operably connected to, and receive blood from, the aortic bifurcation 104. The femoral arteries 110, are operably connected to, and receive blood from, the iliac arteries 112. The veins, which terminate in the superior vena cava 116 and the inferior vena cava 114, carry blood from the tissues of the body back to the right heart, which then pumps the blood through the lungs and back into the left heart. The cerebrovasculature 124 is supplied with blood from major arteries, including the carotid arteries 120, 122, and the vertebral arteries leading, directly or indirectly, from the aortic arch 108.

Pressures within the venous circulation generally average 20 mm Hg or less. The arteries of the circulatory system carry oxygenated blood (not shown) from left ventricle of the heart 102 to the tissues of the body 100. The pressures within the aorta undulate, with a modified triangle waveform, between diastolic pressures of around 80 mm Hg to a systolic pressure of around 120 mm Hg, sometimes called 120/80. A hypotensive person may have arterial pressure lower than 120/80 mm Hg and a hypertensive person may have arterial pressures higher than 120/80 mm Hg. Systolic arterial pressures of about 300 mm Hg, or greater, can occur in extremely hypertensive persons.

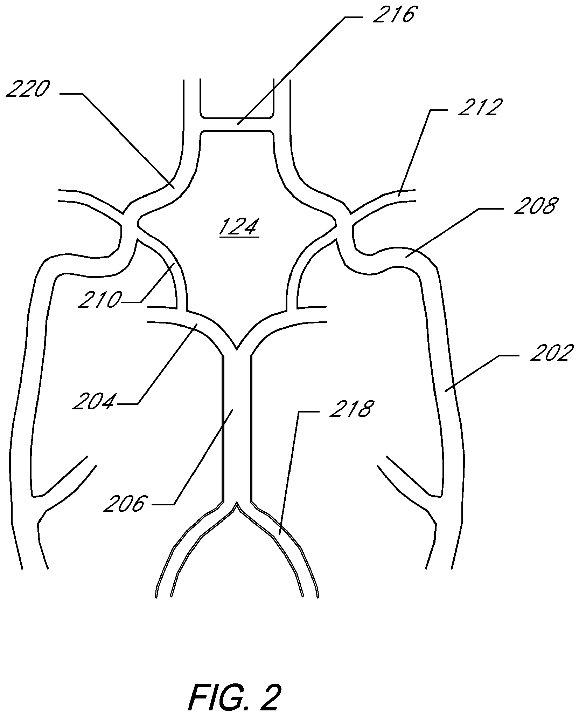

Figure 2 is a schematic frontal illustration, looking posteriorly from the anterior side, of the cerebrovasculature 124. The cerebrovasculature 124 comprises the internal carotid arteries 202, the vertebral arteries 218, the basilar artery 206, the posterior cerebral arteries 204, the posterior communicating arteries 210, the middle cerebral arteries 212, the anterior cerebral arteries 220, and the anterior communicating artery 216.

Referring to Figure 2, the cerebrovasculature is generally symmetric and mirrors about the midline such that the right side is similar to the left side. This provides redundancy and collateral circulation should a blockage occur within the cerebrovasculature, thus ensuring that blood supply still reaches brain tissue. The formation created by the right and left posterior cerebral arteries 204, the right and left posterior communicating arteries 210, the right and left anterior cerebral arteries 220, and the anterior communicating artery 216 is called the circle of Willis. Many neurovascular lesions occur in the region of the circle of Willis, or beyond, so catheter access to the circle of Willis is essential for performing therapy within the neurovasculature. Access to the circle of Willis can be performed through the internal carotid arteries 202 by passing through the tortuous carotid siphon. Access to the circle of Willis can also be achieved through the vertebral arteries and the basilar artery 206, then into the posterior cerebral arteries 204 of the circle of Willis. All access pathways are extremely tortuous and small in diameter and are generally accessed from a catheter passed up the aorta, through the aortic arch 108 and then through the innominate artery or carotid arteries 120, 122.

By accessing the cerebrovasculature through the arterial circulation, the chance of hemorrhage from the catheter insertion site is minimized by use of hemostasis valves built into any catheters, sheaths, hollow needles, or introducers used on the patient. A guidewire is generally of sufficient length that the portion of it that extends outside the body at its proximal end sufficiently to completely pass a catheter thereover with some guidewire extending beyond the proximal end of the catheter. Thus, the guidewire is as long as, or longer than, twice the distance to the treatment site in the patient 100. The most commonly used guidewire diameter ranges from 0.2 mm to 0.5 mm (0.008 inches to 0.018 inches) for cerebral applications and 0.9 mm to 1 mm (0.035 inches to 0.038 inches) or larger for these applications. Guidewires can be PTFE coated to improve lubricity and can have various types of tip configurations including, but not limited to, straight, "J", floppy tip, rigid tip, and the like. Access can be gained through the iliac artery 112 but preferably, the access can be gained through a femoral artery 110. In some embodiments, access can also be gained through the subclavian artery in the shoulder using a somewhat shorter device.

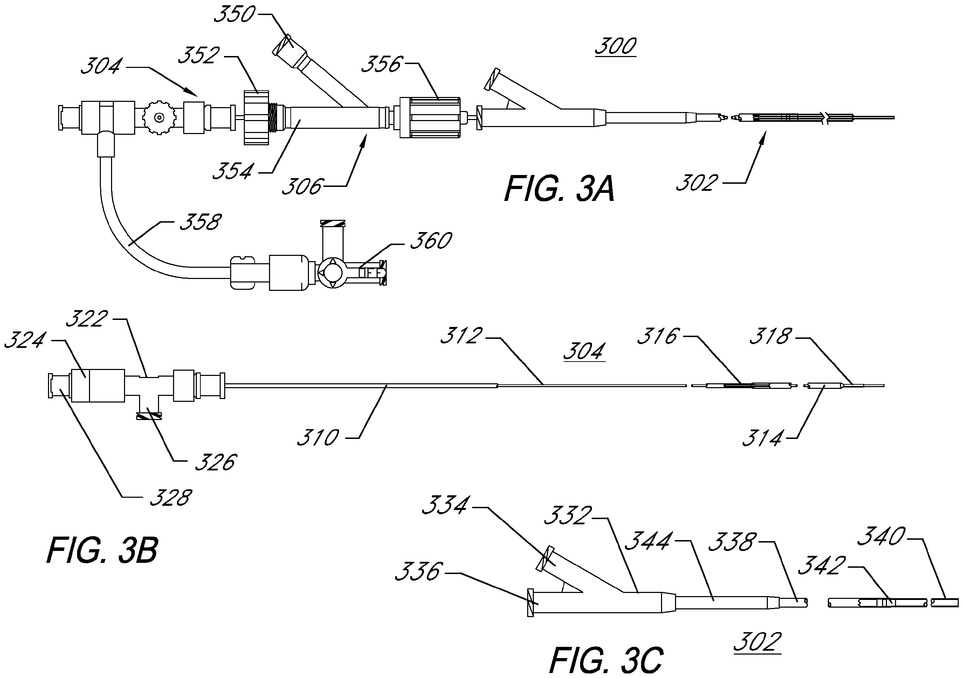

Fig. 3A illustrates one embodiment of an expandable cerebrovascular introduction sheath or guide catheter assembly 300. In this arrangement, the guide catheter assembly 300 can comprise a sheath 302, a dilator 304, and a sheath Y connector 306. The sheath Y connector 306 further comprises a sideport 350, a through port 354 further comprising a hemostasis valve 352, and a male Luer lock 356. The dilator 304 further comprises a purge line 358 terminated by a valve 360.

Referring to Fig. 3A, the Y connector 306 is either permanently affixed, removably attached, or integral to, the proximal through port of the hub on the sheath 302. The Y connector 306 comprises the male Luer lock 356 for this purpose, if removability or exchangeability is required. The purge line 358 is affixed to the valve 360 which can be a stopcock or other type of fluid-tight valve. The purge line 358 is operably connected to central or through lumen of the dilator (not shown), the central or through lumen (not shown) being configured to accept an appropriately sized guidewire. The dilator 304 is slidably inserted through the Y connector 306, which further comprises a hemostasis type valve. The dilator 304 is advanced through the lumen of the sheath 302 until it projects slightly out the distal end of the sheath 302 such that the balloon on the dilator 304 extends slightly beyond the distal end of the sheath 302. The distal, collapsible region of the sheath 302 is now crimped down around the collapsed balloon on the dilator 304 and the dilator 304 is locked in place at the proximal end of the sheath 302. This preparation is generally performed at the manufacturing location, prior to packaging, sterilization, and use. The preparation is preferably performed in a clean room environment with minimal bioburden.

The hemostasis valve 352 preferably comprises ports that further comprise, or are terminated by, hemostasis valves. The hemostasis valves are configured to prevent hemorrhage from, or air intake into, the lumen of the sheath 302. The hemostasis valve 352 can comprise between one and 5 elements to form a seal against nothing inserted into the valve, form a seal against a maximum diameter object inserted through the valve, and form a seal against anything of intermediate size inserted through the valve. The hemostasis valve 352 elements can be fabricated from soft silicone or other elastomer. The hemostasis valve elements can be coated or impregnated with lubricious coatings such as silicone oil or hydrophilic layer. The hemostasis valve 352 elements can comprise duckbill valves, pinhole valves, slit valves, X-slit valves, ring seals, and the like. The hemostasis valve 352 can comprise a Tuohy-Borst valve as all or part of its mechanism.

Fig. 3B illustrates a side view of the dilator 304 configured for use with the expandable guide catheter system 300. The guide catheter dilator 304 comprises a dilator hub 322 further comprising a hemostasis valve 324 and a through port 328. The dilator hub 322 further comprises a sideport 326, which is operably isolated from the through port 328. The dilator 304 further comprises the outer tubing 310, an optional strain relief (not shown), a length of inner tubing 312, and a dilator balloon 314 further comprising a proximal bond 316 and a distal bond 318.

The dilator balloon 314 can be bonded, welded, or otherwise affixed to the dilator catheter tubing 310, 312 by balloon bonds 316, 138, respectively at both ends of the dilator balloon 314. The dilator balloon 314 is fully deflated, flattened, and wrapped around the dilator catheter tubing 312 prior to insertion inside the sheath 302. The proximal end of the dilator balloon 314 is bonded to the exterior of the outer tubing 310. The inner tubing 312 is concentrically disposed within the outer tubing 310. The concentric tubes 310, 312 are configured with a space or annulus between the inside diameter (ID) of the outer tubing 310 and the outside diameter (OD) of the inner tubing such that fluid can pass through this annulus from the sideport 326 to the inside of the balloon 314. The annulus can comprise a radial clearance ranging from about 0.05 mm to 0.6 mm (0.002 inches to 0.025 inches) with a preferred range of 0.1 mm to 0.4 mm (0.005 to 0.015 inches). The distal end of the dilator balloon 314 is bonded to the exterior of the inner tubing 312. Thus, pressurized fluid flowing within the annulus collects and is trapped within the balloon 314, allowing it to pressurize and expand under control of an operator by means of an inflation device or syringe attached to the sideport 326 of the hub 322. The dilator balloon 314 can be bonded, or welded, to the dilator catheter tubing 310 and 312 by application of heat shrink tubing and a distributed heat source such as a radiant heater, laser bonder, forced hot air system, or the like. A catheter rotation device, configured to rotate the catheter about its longitudinal axis, is useful to allow even heating or heat distribution to optimize bond integrity.

Referring to Fig. 3B, the dilator balloon 314 can be fabricated from PET, PETG, polyamide, polyamide, or the like, with wall thickness ranging between 0.03 mm to 0.2 mm (0.001 to 0.006 inches), and can be capable of containing an internal pressure of 10 to 30 atmospheres, or higher. The dilator balloon 314 can be generally filled with incompressible fluid such as, but not limited to, saline, radiographic contrast media, or the like by the operator, through the balloon inflation port 326 integral to, or affixed to, the dilator hub 322.

Fig. 3C illustrates the expandable introduction sheath 302 (in an expanded configuration), which is part of the sheath/dilator system 300 but with the dilator 304 removed. The introduction sheath 302 comprises a sheath hub 332 further comprising the through port 336 and a sideport 334, the proximal non-expandable tubing region 306, the first transition zone 308, the proximal non-expandable region 338, a distal expandable region 340, a transition zone 342, and a strain relief 344.

The sheath hub 332 is affixed to the proximal end of the non-expandable, proximal tubing 338. The distal end of the non-expandable tubing 338 is affixed to the proximal end of the expandable region 340 by the transition zone 342. The hub 332 is also affixed to the proximal end of the strain relief 344, which surrounds and helps reduce stress on the soft proximal tubing 338 where it exits the hard hub 332. The through port 336 is operably connected to a through lumen within the different regions of the sheath tubing 338, 340, 342. There is no operable communication between the through port 336 and the sideport 334. The sideport 334 is operably connected to a separate lumen within the hub 332, which is operably connected to a small, separate lumen or annulus within the proximal sheath tubing 338.

Referring to Fig. 3C, the expandable introduction sheath 302 is illustrated with the distal expandable region 340 and the transition zone 342 having been expanded radially, or diametrically, to their second, larger cross-sectional configuration. Malleable reinforcing structures (not shown), within the transition zone 342 and the distal expandable region 340 maintain the sheath in its second, larger, cross-sectional configuration. Preferably, the malleable elements within the distal expandable region 340 and the transition zone 342 maintain sufficient strength to overcome resilient forces exerted by the polymeric tubing within which the malleable elements are embedded. The malleable elements or reinforcing structure is configured with insufficient strength to overcome the expansion forces of the dilator balloon 314 of Fig. 3B. The reinforcing elements can comprise structures such as, but not limited to, spiral windings of flat or round wire, braided elements of polymeric strands, wire, a mesh structure similar to a stent, a slotted tube with overlapping longitudinally oriented slots, or the like. The length of the sheath tubing from the distal end of the sheath hub 332 to the distal end of the collapsible region 340 can range between about 85-cm and 140-cm with a preferred length of about 95-cm to about 125-cm. The length is long enough to pass through and beyond other commercial guiding catheters and short enough that commercial microcatheters can be passed therethrough and extended beyond the distal end of the collapsible region 340.

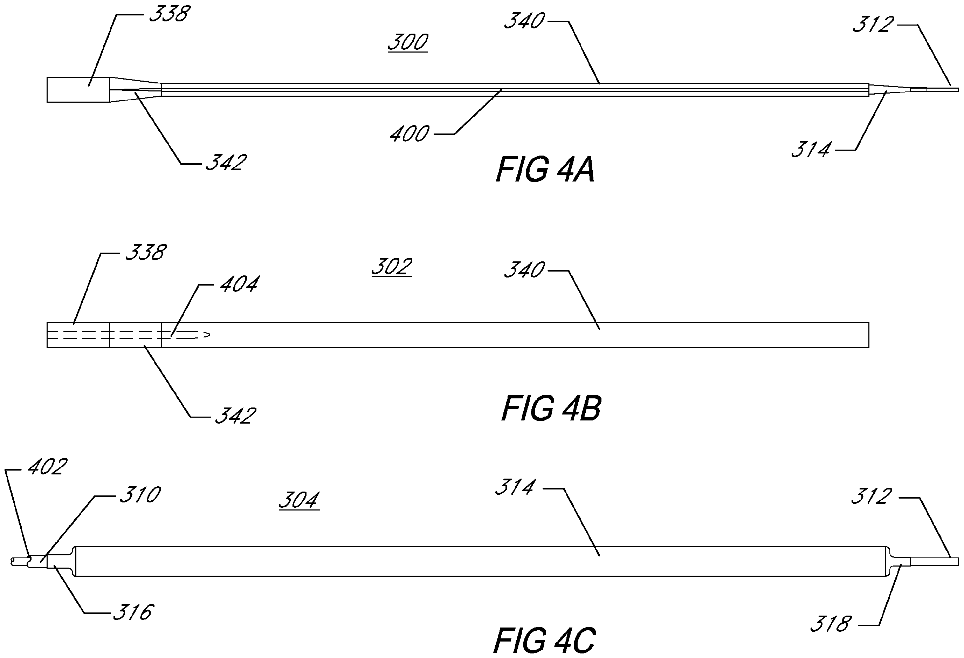

Fig. 4A illustrates the distal end of the expandable guide catheter system 300 in its first, small cross-sectional configuration. The distal end of the guide catheter system 300 comprises the compressible region 340, the transition zone 342, the proximal, non-expandable region 338, the dilator balloon 314, and the dilator inner tubing 312. The compressible region 340 and at least a portion of the transition zone 342 further comprise the longitudinal fold 400.

The compressible region, which is also called the expandable region, 340 and the transition zone 342 are illustrated in their first, smaller cross-sectional configuration. The transition zone 342 forms a taper between the diametrically collapsed expandable region 340 and the larger, proximal, non-expandable region 338. The introducer sheath/dilator assembly 300 of the illustrated embodiment comprises the sheath hub 332 (not shown), which is affixed to the proximal end of the length of proximal non-expandable tubing 338, and the dilator hub 322 (not shown). The compressible region 340 is surrounded by an outer jacket (not shown), a thin, non-compliant, flexible structure, which is sealed to inner layers of the expandable region 340 near the distal end of the collapsible region 340 as well as to the non-collapsible tubing 338 proximal to the transition zone 342. The outer jacket (not shown) is delaminated from the inner components of the expandable region 340 such that pressurization of this delaminated region causes collapse of the inner components. The outer jacket (not shown) is extremely strong and generally, or substantially inelastic at its rated pressure, and is fabricated from materials such as, but not limited to, polyimide, polyamide, polyester, irradiated and cross-linked polyethylene, or the like. The ends of the outer jacket (not shown) are bonded, welded, or otherwise sealed to the inner layers of the sheath tubing to create a fluid-tight seal that can be folded and straightened out multiple times, especially at the distal end, without a failure of the fluid-tight seal. In other embodiments, the outer jacket (not shown) can comprise two layers, sealed to each other at the ends that can form a balloon to inward compress the inner layer and the inner layers of the expandable region 340. The double wall structure can be advantageous in that the bond seals at the ends may be easier to maintain during folding and unfolding. The dilator balloon 314 runs the entire length and slightly beyond the extents of the expandable region 340. Outer jacket welding to the sheath tubing, dilator balloon 314 to dilator tubing, or both, can be accomplished using shrink tubing to compress the weld area under heat, a means for imposing heat on the weld area, including, but not limited to, laser, forced air heater (e.g. hot box), radiant heater, or the like. A turntable or other rotation system can be used to rotate the sheath or catheter shaft about its longitudinal axis to improve heat distribution, especially when the heat is in the form of a jet or source flow such as from a "hot box" or laser. The length of the jacket or balloon bond can range from about 0.1 mm to 6 mm (0.005 to 0.250 inches) with a preferred range of about 1 mm to 4 mm (0.040 to 0.150 inches).

The materials used for construction of the inner layers of the distal, collapsible region 340 and the transition zone 342 include but are not limited to, Hytrel, polyethylene, PET, LDPE, HDPE, HDPE/LDPE blends, PFA, FEP, PTFE, polyimide, Pebax, and the like. Hytrel is a thermoplastic elastomer that can be more easily welded to PET, a material suitable for making the outer jacket (not shown). Pebax is a block polyamide that is more suitable for bonding or welding to outer jacket materials made from Nylons or other polyamides.

Fig. 4B illustrates the distal end of the expandable introduction sheath 302, which is part of the sheath/dilator system 300, but with the dilator 304 removed. The expandable region 340 and the transition zone 342 have been fully expanded to their second, larger, cross-sectional configuration. The introduction sheath 302 comprises the sheath hub 332 (not shown), the proximal non-expandable tubing region 338, the transition zone 342, and the expandable region 340. The proximal non-expandable tubing 338 further comprises a separate inflation lumen 404 that runs longitudinally and operably connects a sheath deflation port on the sheath hub 322 to the delaminated region between the outer jacket and the inner parts of the expandable region 340.

Referring to Fig. 4B, the expandable region 340, in some embodiments, can comprise shape memory elements (not shown) fabricated from nitinol, which is configured with an austenite finish temperature in excess of body temperature (normally around 37 degrees centigrade). Thus, the expandable region 340 can be heated by application of electricity to generate resistive heating and temperature increase to above the austenite finish temperature. A suitable austenite finish temperature can range from 38 to 50 degrees centigrade. Such heating can be performed at the conclusion of the procedure, following removal of any therapeutic or diagnostic instruments from the center of the sheath. The sheath will generally be within the blood stream and not touching any vascular walls. Furthermore, flowing blood can disperse heat generated by the resistive heating elements so as to minimize localized heating damage effects to the body. The shape memory elements can be heat set to a collapsed, small diameter configuration to which they will be biased following application of resistive heating. The reinforcing structures can be configured as a braid, a spiral winding, a woven mesh, a slotted tube, or the like. The reinforcing structures can be heat set in a collapsed, or small, initial diameter configuration and then be cooled to below martensite finish temperature, at which point the reinforcing structures can be expanded for coating with a polymer or other suitable manufacturing process. The reinforcing structures, which can be fabricated from malleable stainless steel in the preferred embodiment, or from nitinol, as described herein, can be configured as spiral windings, stent-like structures, braids, or the like.

The proximal sheath tube 338, which is affixed at its proximal end to the sheath hub (not shown), can comprise one or two layers of mesh reinforcement and a spring-coil reinforcement. The distal sheath tube 340 and the transition zone 342 can further comprise a malleable coil and, optionally, the mesh reinforcement. The entire sheath tube, which comprises a central lumen (not shown), comprises an approximately constant inner diameter along its entire length. The approximately constant diameter is beneficial in that objects of large diameter can be inserted and advanced completely from the proximal end and out the distal end of the sheath 302.

In one embodiment, an inner sheath layer is first laid down over a PTFE-coated stainless steel mandrel (not shown). The sheath inner layer can be preferably fabricated from lubricious materials such as, but not limited to, polyethylene, HDPE, LDPE, blends of HDPE and LDPE, PTFE, FEP, PFA, Hytrel, Pebax, or the like. The sheath inner layer can also be coated, on its inner surface, with friction retarding materials such as, but not limited to, silicone oil, polyurethane-based hydrophilic slip coating materials, and the like. The optional mesh layer is next applied over the inner layer. The coil reinforcement layers and can, next, be applied over the mesh. In other embodiments, a second layer of mesh can optionally be applied over the coil. The second layer of mesh can have different properties from the inner layer of mesh, including different filament diameter, filament count, number of picks, and filament density or angle. Finally, an outer layer of polymeric material can be applied over the reinforcement, after which shrink tubing can be placed around the entire structure and heated to shrink, melt, fuse, and bond the inner layer to the outer layer while sandwiching the reinforcing layers therebetween. The sheath inner layer can have a wall thickness ranging between about 0.03 mm to 0.3 mm (0.001 and 0.010 inches) with a preferred range of about 0.04 mm and 0.2 mm (0.0015 and 0.006 inches). The sheath outer layer can have a wall thickness ranging between about 0.03 mm and 0.3 mm (0.001 and 0.010 inches) with a preferred range of about 0.04 mm to 0.2 mm (0.0015 to 0.006 inches).

The mesh can be formed from a braid, weave, knit or other structure formed into a tubular cross-section. The mesh can be fabricated from flat, rectangular, or round strands. The mesh can be fabricated from polymers such as, but not limited to, polyethylene naphthalate (PEN), PET, polyamide, polyimide, or the like. The mesh can also be fabricated from metals such as, but not limited to, malleable stainless steel, spring stainless steel, nitinol, titanium, cobalt nickel alloy, tantalum, gold, platinum, platinum alloy, and the like. The lateral size of the strands of the mesh can range between 0.03 mm and 0.3 mm (0.001 and 0.010 inches) in at least one dimension. The number of ends of the mesh can range between 2 and 50.

The construction of the distal sheath tube 340 can comprise a coil of wire with a wire diameter of 0.03 mm to 1 mm (0.001 to 0.040 inches) in diameter and preferably between 0.05 mm and 0.3 mm (0.002 and 0.010 inches) in diameter. The coil can also comprise a ribbon wire or a flat wire that is 0.03 mm to 0.3 mm (0.001 to 0.010 inches) in one dimension and 0.1 mm to 1 mm (0.004 to 0.040 inches) in the other dimension. Preferably, the flat wire is 0.03 mm to 0.1 mm (0.001 to 0.005 inches) in the small dimension, generally oriented in the radial direction of the coil, and 0.1 mm to 0.5 mm (0.005 to 0.020 inches) in width, oriented perpendicular to the radial direction of the coil. The pitch of the coil, which is related to the spacing between coil turns can range from about 0 to about 5 times the ribbon width or wire diameter. Preferably, some space exists between the coil turns to permit bonding between the outer layer and the inner layer so a preferred spacing is between 0.5 and 4 times the width of the ribbon. The outer layer of polymeric material can have a wall thickness of 0.03 mm to 0.5 mm (0.001 to 0.020 inches) and the inner layer has a wall thickness of between 0.03 mm and 0.3 mm (0.001 and 0.010 inches). The wire used to fabricate the coil can be fabricated from annealed materials such as, but not limited to, gold, stainless steel, titanium, tantalum, nickel-titanium alloy, cobalt nickel alloy, and the like. The wire is preferably fully annealed. The wires can also comprise polymers or non-metallic materials such as, but not limited to, PET, PEN, polyamide, polycarbonate, glass-filled polycarbonate, carbon fibers, or the like. The wires of the coil reinforcement can be advantageously coated with materials that have increased radiopacity to allow for improved visibility under fluoroscopy or X-ray visualization. The radiopaque coatings for the coil reinforcement may comprise gold, platinum, tantalum, platinum-iridium, and the like. The mechanical properties of the coil are such that it is able to control the configuration of the fused inner layer and the outer layer.

When the distal region 340 is folded to form a small diameter, the polymeric layers, which can have some memory, do not generate significant or substantial spring-back. The sheath wall is preferably thin so that it any forces it imparts to the tubular structure are exceeded by those forces exerted by the malleable distal reinforcing layers. Additionally, a peel away, slide away, or otherwise removable protective sleeve (not shown) is useful but not necessary to maintain the collapsed sheath configuration.

It should be appreciated that modifications thereof can be used to provide an expandable region of the catheter with an initial small cross-sectional diameter. By unfolding the distal region 340, the diameter of the distal region can be increased to a larger diameter. In the smaller folded configuration, the malleable structures described above can maintain the distal region in the smaller folded configuration. In other embodiments, an external structure can maintain the sheath in the folded configuration. In this smaller folder configuration it has been noted that the flexibility of the catheter (e.g., the ability of the catheter to navigate the carotid siphon) is increased. When the catheter is unfolded and expanded, the malleable structure can reform to the larger unfolded diameter and to the shape of the anatomy in which the sheath his placed. In the unfolded configuration, the malleable structures provide hoop strength maintain the patency of the lumen.

In other embodiments, the exterior of the sheath, and optionally the internal lumen of the sheath, can be coated with a lubricious coating comprising materials such as, but not limited to, silicone oil or a hydrophilic hydrogel comprising polyethylene glycol, polyether polyurethane, or the like. Other coatings can include antimicrobial coatings such as those fabricated from silver azide or anticoagulant coatings such as those comprising heparin.

Fig. 4C illustrates the distal end of a sheath dilator 304 comprising the dilator outer shaft 310, the dilator hub 322, the dilator balloon 314, an optional distal fairing (not shown), and the dilator inner tubing 312. The dilator balloon 314 comprises proximal and distal neck down regions, 316 and 318, respectively. The inflation annulus 402 is that region between the concentrically disposed outer shaft or tubing 310 and the inner shaft or tubing 312 of the dilator.

The dilator balloon 314 is affixed to the outer dilator shaft 310 at the proximal neck down region 316 and to the dilator inner tubing 312 at the distal neck down region 318 using adhesives, welding, or a combination thereof to form balloon bonds 316, 318. The dilator balloon 314 can be an angioplasty type balloon fabricated from material such as, but not limited to, PET, polyimide, polyamide, reinforced polymers, or the like. The dilator balloon 314 can be configured to generate pressures ranging up to about 25 or 30 atmospheres when filled with pressurized liquids such as, but not limited to, radiopaque dye contrast media, saline, Ringer's lactate, and the like. The dilator balloon 314 comprises a flat length at least as long as the combined length of the sheath expandable distal region 340 and the transition zone 342, and is preferably somewhat longer to facilitate manufacturability and reliability. The dilator balloon 314 can comprise an inflated diameter approximately equal to or slightly greater, for example about 0.2 mm to 0.3 mm (0.5 to 1 French) larger in diameter, than that of the fully expanded distal region 340 of the sheath. The balloon 314 can comprise wall thicknesses ranging from 0.01 mm to 0.1 mm (0.0005 to 0.005 inches) and preferably ranging between about 0.02 mm and 0.05 mm (0.0007 and 0.002 inches). The flat length of the balloon 314 is advantageously about at least 1-cm longer than the combined length of the transition zone 342 and the collapsible region 340. The flat length of the balloon 314, therefore can range between about 9-cm to about 31-cm. Note that the distal fairing (not shown) is affixed proximate the distal tip of the dilator 304, and is preferably affixed, at least in part to the dilator inner tubing 312 where it projects distally from the distal balloon bond 318. The distal fairing (not shown) is beneficially fabricated from soft elastomeric materials such as silicone elastomer or thermoplastic elastomer, and expands and folds distally off the shoulders of the balloon 314 such that when the balloon 314 is deflated, the fairing (not shown) returns to a small diameter that can be withdrawn proximally through the lumen of the sheath 304. The distal fairing (not shown) can be fabricated from elastomeric materials such as, but not limited to, thermoplastic elastomer, silicone elastomer, polyurethane elastomer, Hytrel elastomer, and the like.

The dilator 304 is slidably disposed within the central lumen of the sheath 302 and further comprises an expandable dilator 314 such as, but not limited to, an angioplasty type balloon, malecot, reverse collet, or other device capable of expansion to approximately 0.2-mm (0.5 French), or greater, larger than the diameter of the sheath. The balloon 314 can be inflated through the inflation lumen within the catheter shaft, which is operably connected, at its proximal end, to a dilator hub or inflation port. Following inflation, which expands the distal end of the sheath, the dilator expansion element, such as the balloon (not shown), can be deflated or collapsed, following which it can be removed from the sheath 302 along with the nose cone or distal fairing (not shown).

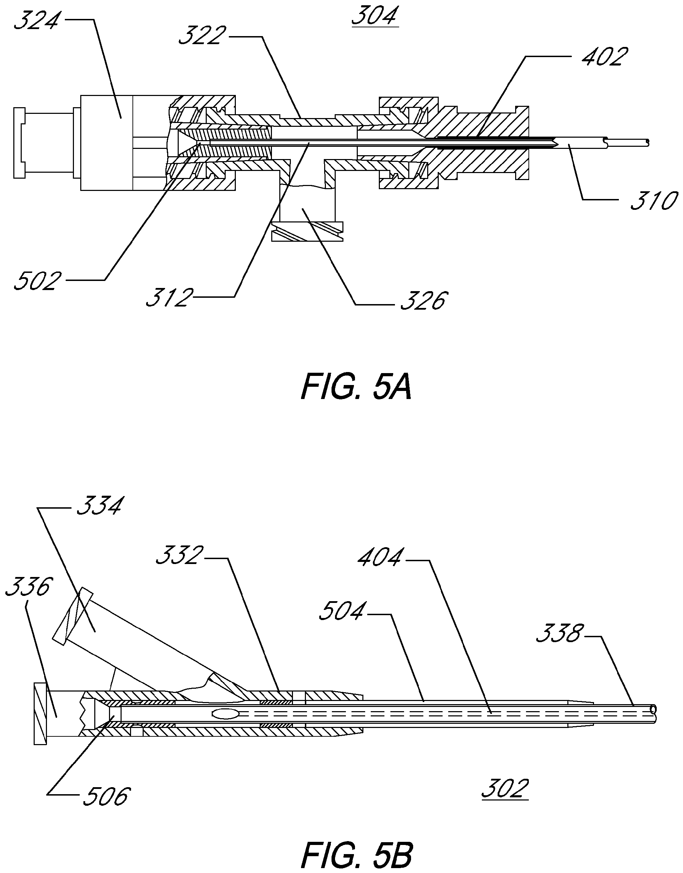

Fig. 5A illustrates the proximal end of the dilator 304. The dilator comprises the dilator hub 322, the outer tubing 310, and the inner tubing 312. The dilator hub 322 further comprises the inflation port 326, the central lumen 502, the hemostasis valve 324, and the inflation annulus 402 between the inner tubing 312 and the outer tubing 310.

Referring to Fig. 5A, the dilator hub 322 is affixed, or coupled, to the proximal end of the outer tubing 310 and can further comprise a strain relief (not shown) surrounding the outer tubing 310 where it exits the distal end of the hub 32. The dilator hub 322 is affixed, at its proximal end, or integral to, the hemostasis valve 324, which can further comprise a female Luer lock connector at its most proximal end. The lumen of the hemostasis valve 324 is operably connected to the central lumen 502 of the hub 322, which is operably connected to the central lumen of the inner tubing 312. The inflation port 326 is beneficially terminated with a female Luer lock connector suitable for attachment to a commercial balloon inflation device or syringe (not shown). The inflation port 326 is operably isolated from the central lumen 502 but is operably connected to the inflation annulus 402. Thus, pressurized fluid, preferably liquid, infused into the inflation port 326 is routed into the annulus 404, where it is transmitted along the length of the dilator 304 to a region under the dilation balloon 314 where it exits to pressurize, or depressurize the dilator balloon 314. The dilator hub 322 and its attached valves can be fabricated from materials such as, but not limited to, ABS, PVC, polyurethane, polycarbonate, and the like. The dilator hub 322 further comprises a purge line 358 (shown in Fig. 3A), which is operably connected to the central lumen 502 and is used to remove air from the central lumen 502 of the dilator 304.

Referring to Fig. 5A, the dilator hub 322 is affixed and sealed to the proximal end of the inner dilator tubing 312. The distal end of the inner dilator tubing 312 extends to the distal end of the dilator. The inner lumen 502 is sized to accept commercially available guidewires and can range in size from about 0.3 mm to 1 mm (0.010 inches to 0.042 inches) in diameter with a preferable diameter of about 0.5 mm (0.018 inches) such that it can slidably accept a 0.4 mm (0.014-inch) diameter guidewire. The overall working length of the dilator from the proximal end of the dilator hub 322 to the distal end of the inner tubing 312 can range between about 90 and 150 cm.

Fig. 5B illustrates the proximal end of the two-way expandable, or collapsible, sheath assembly 302 following removal of the dilator 304. The sheath assembly 302 further comprises the expandable region 340 (not shown), the transition zone 342 (not shown), the proximal non-expandable sheath tubing 338, and the sheath hub 332 further comprising the sheath collapse side port 334, the central through port 336, and the central lumen 506.

The sheath hub 332 can be fabricated from the same, or similar materials, as those used for fabrication of the dilator hub 322. The sheath hub 332 is affixed to the proximal end of the proximal sheath tubing 338 and can further be affixed to an optional strain relief 504 to ease the stress on the relatively soft sheath proximal tubing 338 as it exits the distal end of the relatively hard sheath hub 332. The central through port 336 is operably connected to the central lumen 506, also called a through lumen since it extends to and out the distal end of the sheath collapsible region 340. The sheath collapse port 334 is operably connected to a separate pressurization lumen 404 that is fluidically isolated from the central lumen 506 and which runs at least to the distal end of the proximal sheath tubing 338. The pressurization lumen 404 can be located as a bump on the exterior of the proximal tubing 338, it can be coextruded entirely within a wall of the proximal tubing 338, or a combination thereof.

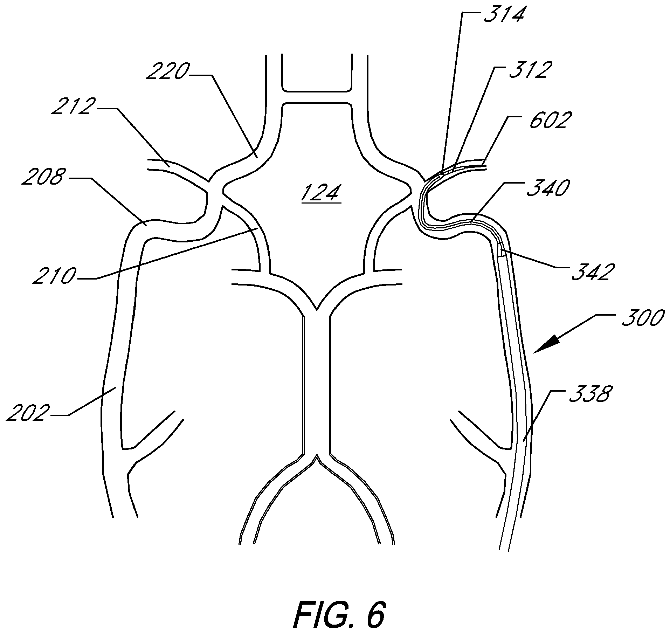

Fig. 6 illustrates a schematic of the cerebrovasculature 124 comprising the internal carotid arteries 202 further comprising the carotid siphons 208, the posterior communicating arteries 210, the middle cerebral arteries 212, the anterior cerebral arteries 220, and the anterior communicating artery 216. An expandable guide catheter 300 has been advanced through the left carotid artery such that its proximal, non-expandable tubing 338 and the transition zone 342 reside proximal to the carotid siphon 208. The distal expandable region 340 enclosing the inner dilator tube 312 and the dilator balloon 314 are highly flexible, torqueable, and pushable, and have navigated the carotid siphon 208 and the proximal part of the middle cerebral artery 212 over a guidewire 602. In this embodiment, the guidewire is a 0.4 mm (0.014-inch) diameter guidewire.

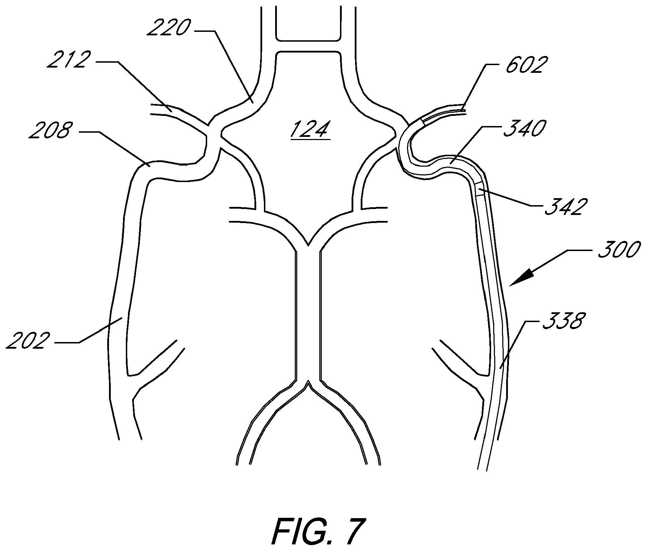

Fig. 7 illustrates the cerebrovasculature 124 of Fig. 6 with the expandable guide catheter 300 having been dilated at its distal end and the dilator removed leaving the guidewire 602 in place. The transition zone 342 is no longer tapered but is substantially cylindrical as is the expanded distal region 340 such that the distal region retains an inner diameter substantially the same as that of the proximal tubing 338. Illustrated are the internal carotid arteries 202, further comprising the carotid siphons 208, the posterior communicating arteries 210, the middle cerebral arteries 212, the anterior cerebral arteries 220, and the anterior communicating artery 216.

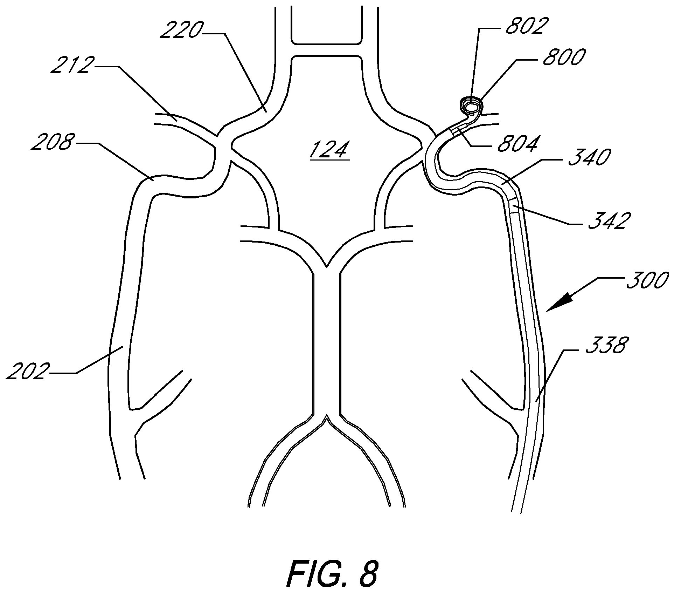

Fig. 8 illustrates the cerebrovasculature 124 of Fig. 6 with the addition of a berry aneurysm 800 located on a middle cerebral artery 212. The expanded guide catheter 300 is being used to guide a pusher catheter 804 such that the pusher catheter 804 can deploy an embolic coil 802 within the aneurysm 800. The guidewire 602 of Fig. 6 has been removed. Following completion of the procedure, all internal catheters, e.g. the pusher catheter 804, are removed. The distal region 340 is next collapsed by pressurization of the region between the outer jacket and the inner portions of the distal region 340. Following evacuation of the collapsing pressure, the collapsed distal region 340 can now be removed from the cerebrovasculature without the potential for damage to sensitive vessel walls.

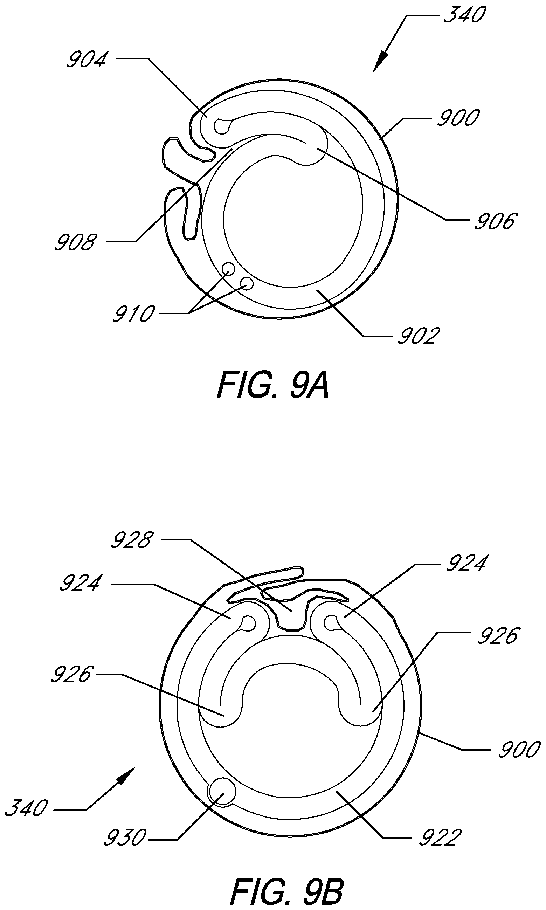

Fig. 9A illustrates a lateral cross-section view of a distal region 340 of an expandable arterial sheath comprising an outer jacket 900, an inner wall 902, a single longitudinally extending fold 908 further comprising an outside edge 904 and an inside edge 906, and a plurality of electrical conductors running axially through the inner wall 902. With a small diameter distal section 340 and a relatively thick wall 902, a single fold 908 is the one structure to create during manufacturing. The sheath inner wall 902 further comprises an optional electrical bus 912 fabricated from stainless steel, silver, copper, or other conductor metal for use in transmitting electrical energy from the sheath hub (not shown) to distal regions of the sheath for purposes such as resistive heating, steering, or the like. The space between the non-distensible outer jacket 900 and the inner wall 902 is pressurized by the operator when re-collapse of the inner wall 902 is desired. The outer jacket 900 is folded in this illustration but maintains an unstretched circumference approximating that of the proximal portion of the sheath tubing 338.

Fig. 9B illustrates another embodiment of a lateral cross-section of a distal region 920 of an expandable arterial sheath comprising the outer jacket 900, an inner wall 922 further comprising a double longitudinally extending fold 928. The double fold 928 further comprises two outside edges 924 and two inside edges 926, which form longitudinal creases in the inner wall 922. When the diameter of the sheath increases, it becomes advantageous to form a plurality of folds in the inner wall 922. A double fold can allow a larger diameter sheath to fold into a collapsed diameter more efficiently. The sheath inner wall 922 further comprises an optional balloon inflation lumen 930 for use in transmitting fluidic pressure or energy from the sheath hub to distal regions of the sheath wherein a balloon may be affixed or for sheath wall re-collapse. The diameter of the balloon inflation lumen 930 can range between about 0.1 mm to 0.5 mm (0.004 to 0.020 inches). In other embodiments, the number of folds can range in number between 3 and 10. The space between the non-distensible outer jacket 900 and the inner wall 922 is pressurized by the operator when re-collapse of the inner wall 922 is desired. The outer jacket 900 is folded in this illustration but maintains an unstretched circumference approximating that of the proximal portion of the sheath tubing 338.

The distal sheath tubing 340 is folded longitudinally in a carefully predetermined pattern comprising between one and four exterior fold edges, wherein the folds extend all the way from the proximal end of the transition zone 342 to the distal end of the distal sheath tube 342. The optional distal fairing (not shown) is configured to cover the distal exposed edge of the distal sheath tube 340 to provide a smooth taper against which the sheath system 300 can be advanced into the cerebrovasculature. The distal fairing can also be configured as a bump that leads the distal end of the sheath, is affixed to the dilator tubing, but does not cover the distal end of the collapsed sheath tubing. The distal fairing can preferably be fabricated from soft elastomeric materials to permit flexibility along its length. Such materials can include Hytrel, silicone elastomer, polyurethane, Pebax, and the like.

It should be appreciated in the embodiments described above that the longitudinal folds of Figures 9A and 9B or modifications thereof can be used to provide an expandable region of the catheter with an initial small cross-sectional diameter. By unfolding the distal region 340, the diameter of the distal region can be increased to a larger diameter. In the smaller folded configuration, the malleable structures described above can maintain the distal region in the smaller folded configuration. In other embodiments, an external structure can maintain the guide catheter or sheath in the folded configuration. In this smaller folder configuration it has been noted that the flexibility of the catheter (e.g., the ability of the catheter to navigate the vertebral and basilar arteries) is increased. When the guide catheter is unfolded and expanded, the malleable structure can reform to the larger unfolded diameter and to the shape of the anatomy (e.g., the carotid siphon 208) in which the sheath 300 his placed. In the unfolded configuration, the malleable structures provide hoop strength maintain the patency of the lumen. In certain embodiments, the lumen can have a diameter ranging from about 1.7 mm to 2.1 mm (0.068 to 0.082 inches) for a sheath with about 2.3 mm (7 French) outside diameter.

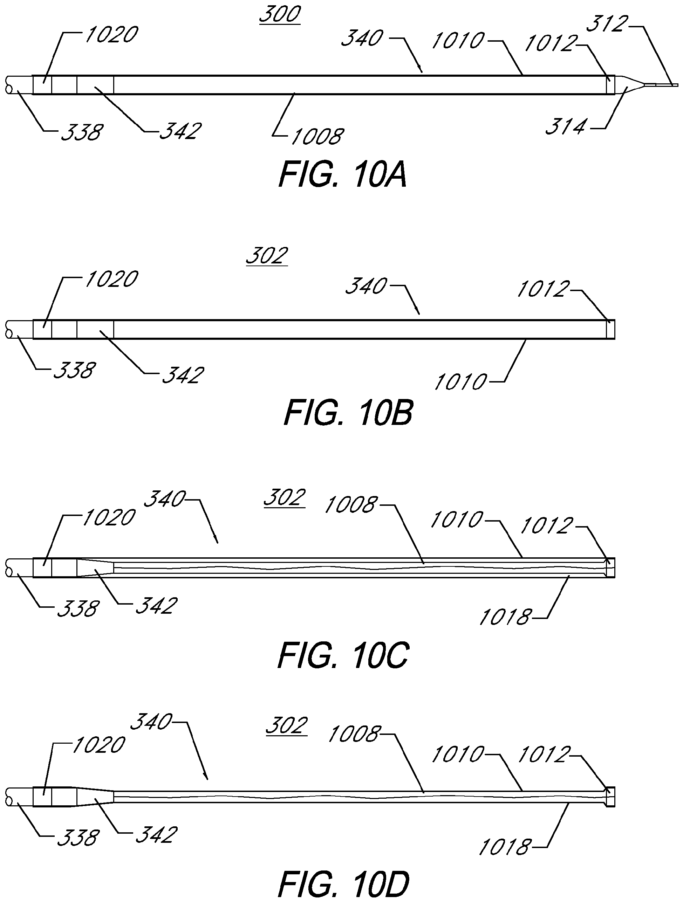

Fig. 10A illustrates the distal end of the expandable, re-collapsible sheath 302 of the guide catheter 300, in its second, radially expanded configuration with the inflated dilator 304 still in place. The outer jacket 1010 has expanded and unfolded with the sheath tubing 1008, 342 to approximate its maximum profile. The dilator 304 and its dilator balloon 314 remain in place within the sheath 302. The sheath tubing 338, 342, 340 retains a generally continuous profile and substantially continuous internal lumen (not shown) of substantially the same size throughout, although some minor distortions of the distal collapsible region 340 can occur.

Referring to Fig. 10A, the re-collapsible introducer sheath 300 comprises the dilator 304 further comprising a dilator balloon 314 and a length of inner dilator tubing 312, a proximal, non-collapsible sheath tube 338, the transition zone 342, the distal, collapsible region 1008, an outer pressurization jacket 1010, outer pressurization jacket to sheath proximal bond 1020, and an outer pressurization jacket to sheath distal bond 1012.

Referring to Fig. 10A, the sheath and dilator system 300 comprises the external pressurization jacket 1010, which is affixed and sealed to the sheath tubing 338 and 1008 at the proximal and distal ends, respectively. A lumen 404 (Fig. 4B) operably connects the collapse port 334 on the sheath hub 332 (Fig. 3C) to the gap 1018 between the outer pressurization jacket 1010 and the sheath tubing 1008. The proximal end of the external pressurization jacket 1010 is preferably affixed to the sheath tubing 338 in the proximal non-collapsible region 338 or the transition zone 342. The external pressurization jacket 1010 can also be operably connected to, or affixed to the sheath hub 332 such that an annulus lumen exists between the inside of the jacket 1010 and the outside of the sheath tubing 338, allowing pressurized fluid to flow to and from the gap between the jacket 1010 and the sheath tubing 338. The pressurization jacket 1010 can be fabricated from foldable materials that are substantially non-distensible or non-elastic such as, but not limited to, polyester, polyimide, polyamide, irradiated polyethylene, and the like. The wall thickness of the outer jacket 1010 can range between 0.003 mm and 0.1 mm (0.0001 inches and 0.005 inches) with a preferred wall thickness range of 0.005 mm and 0.05 mm (0.0002 and 0.002 inches). Such structures for the pressurization jacket 1010 are substantially size constrained or limited and do not expand excessively in their exterior dimensions since the pressurization jacket 1010 is substantially non-compliant.

In other embodiments, the outer jacket 1010 can comprise a double layer of material such as a double layer of polyester (PET) with wall thickness ranging between 0.004 mm and 0.1 mm (0.00015 inches and 0.005 inches) with a preferred wall thickness range of 0.005 mm and 0.05 mm (0.0002 and 0.002 inches). The double layer is advantageous because it permits a strong pressure seal to be created in a situation where such a seal might not otherwise be possible given the dissimilar nature of the material of the outer jacket 1010 and the sheath tubing 338, 342, 1008. Further, the double wall can be bonded or terminated at a point distal to the most distal end of the sheath to allow for full sheath refolding and re-collapse when the pressurization jacket 1010 is pressurized. The sheath tubing 338, 342, 1008 also, preferably comprises a malleable metal reinforcement layer embedded therein that controls the shape of the sheath tubing when not being moved by the dilator 304 or pressurization of the region interior to the outer jacket 1010. Pressurization of the collapsing annulus 1018 can be performed using a syringe, PTCA inflation device, or the like at pressures ranging from about 1 to 30 atmospheres (1,013 - 30,4 bar) and preferably between about 4 to 6 atmospheres, (4,05 - 6,08 bar) using non-compressible fluids such as saline, water, or radiopaque contrast media injected into the sheath hub collapsing port 334.

In some embodiments, the outer pressurization jacket 1010 can be welded to the outside of the sheath tubing 338 using heat and pressure. Materials for use in the sheath tubing 338 and 1008 are generally chosen to optimize the heat weld between the pressurization jacket 1010 and the sheath tubing 338 and 1008. The pressure can be applied to the assembly by fabrication over an inner mandrel and application of heat through a heat shrink band fabricated from FEP, PTFE, or the like. The distal bond between the sheath tubing 1008 and the pressure jacket 1010 is preferably short, between about 1mm and 10mm long, for example, and is strong, durable, and flexible. The distal bond between the sheath tubing 1008 and the outer jacket 1010 needs to be leak free at the rated pressure following extended periods of folding through sharp bends, sterilization, shipping, and storage, the storage and shipping often occurring at extremes of high or low temperature. In an exemplary embodiment, the inner sheath layer is fabricated from Hytrel with a hardness of about 55D and a wall thickness of about 0.03 mm to 0.08 mm (0.001 to 0.003 inches) while the outer layer 1008 of the foldable region is fabricated from Hytrel with a hardness of about 40D and has a wall thickness of about 0.1 mm to 0.3 mm (0.004 to 0.010 inches). The pressure jacket 1010 can be fabricated from PET with a wall thickness of about 0.005 mm to 0.01 mm (0.0002 to 0.0004 inches). In some embodiments, the inner sheath layer can be fabricated from PET with a wall thickness of about 0.01 mm to 0.05 mm (0.0005 to 0.002 inches), instead of, or in addition to, the inner Hytrel 55D layer. In yet another embodiment, the inner layer is Hytrel but a short layer of very thin PET, about 0.006 mm to 0.008 mm (0.00025 to 0.0003 inches) thick, is used to reinforce the Hytrel sheath near its distal end such that the short PET reinforcing layer is about 0.5 to 1.0 cm long and covers the distal end of the reinforcement layer.

Fig. 10B illustrates the distal portion of the sheath 302 having been expanded by the dilator 304 of Fig. 10A, and the dilator 304 subsequently removed. The sheath distal portion comprises the expandable region 340, the transition zone 342, the proximal tubing 338, the outer jacket 1010, the proximal jacket bond 1020 and the distal jacket bond 1012. The outer jacket 1010 is distended to substantially its maximum diameter and does not expand further.

Fig. 10C illustrates the distal end of the expandable guide catheter sheath 302 with the dilator 304 (see Fig. 10A) removed and the space 1018 between an outer jacket 1010 and the introducer sheath 1008 pressurized to collapse the introducer sheath distal tube 1008 to its third, radially collapsed configuration. The gap 1018 between the outer jacket 1010 and the sheath tubing 1008 is visible in this illustration. The inner tubing of the transition zone 342 tapers to the smaller diameter of the collapsed distal, collapsible region 1008.

The outer jacket 1010 can be a single layer or it can comprise a double layer that can be everted, adhesively adhered, or welded to itself at the distal end. The double layer outer jacket 1610 has the advantage of providing a very strong bond and, thus improved inflation reliability, as well as the ability to completely collapse the collapsible sheath tubing 1008 substantially all the way to, and including, the distal end of the collapsible sheath distal tubing 1008.

In other embodiments, the expandable region 340 is re-collapsed to its third, smaller cross-sectional configuration by application of heat to a shape-memory reinforcement embedded within the expandable region. The expandable region 340 can be made to uniformly compress to a smaller diameter, or it can be made to fold into any of a variety of cross-sectional patterns exhibited by a tube that is folded along longitudinally disposed folds. In the embodiments where uniform reduction in cross-sectional shape is imparted, the reinforcement can comprise a braid that elongates longitudinally when it reduces its diameter. The polymeric material 1008 of the expandable region 340 is preferably elastomeric and comprises materials such as, but not limited to, polyurethane, thermoplastic elastomer, silicone elastomer, and the like. The interior wall of the lumen (not shown) of the expandable region is advantageously coated with a layer of high lubricity and low friction to facilitate catheter or device introduction therethrough without hang-up.

Fig. 10D illustrates the distal end of the expandable guide catheter sheath 302 following completion of the collapsing step. In Fig. 10D, the fluid has been withdrawn from the gap 1018 thus causing the outer jacket 1010 to become flaccid and at least partially collapse, thus facilitating removal of the now smaller diameter guide catheter sheath 302 from a patient.

In other embodiments, the outer pressure jacket 1010 is affixed to the sheath outer surface by adhesives or welding such that a region of unadhered pressure jacket 1010 is diposed longitudinally along the sheath tubing 1008. Pressurization of the region between the pressure jacket 1010 and the sheath tubing 1008 causes only the unadhered region to expand and force the sheath tubing 1008 to deflect inward to form a "U-shaped" cross-section which is more controlled and forms a smaller crosss-section than would be possible with a pressure jacket 1010 completely detached from the sheath tubing 1008 in the central region but bonded only at the ends. Evacuation of the fluid resident between the pressure jacket 1010 and the sheath tubing 1008 is beneficial to provide a minimum sheath cross-section for removal from the patient. In certain embodiments, the unadhered region of the pressure jacket 1010 can range from about 20% of the sheath circumference to about 50% of the sheath circumference.

In yet other embodimens, the pressure jacket 1010 can surround a side balloon (not shown) that is disposed longitudinally along one side of the sheath tubing 1008 such that the side balloon can be inflated under pressure to collapse the sheath tubing 1008 along only the region adjacent to the side balloon. The side balloon length is beneficially about slightly longer than region of re-collapse, which is generally the same as the expandable region of the sheath. The substantially non-compliant pressure jacket 1010 serves to provide counterforce to the substantially non-compliant side balloon to permit the side balloon to "punch" a "U-shaped" cross-section into the sheath tubing. The side balloon is operably and fluidically connected to a re-collapse inflation port near the distal end of the sheath by a lumen or annulus comprised by the sheath structure. The side balloon can comprise a distal seal or it can be formed with a completely closed end requiring no seals or bonds. The side balloon and the pressure jacket 1010 can be fabricated from the same materials disclosed herein for use in the pressure jacket 1010. The pressure jacket 1010 can serve as a friction, puncture, or damage shield for the side balloon thus increasing the robustness of the system. The side balloon can control re-folding or re-collapse in the same way as the partially bonded pressure jacket 1010 disclosed herein.

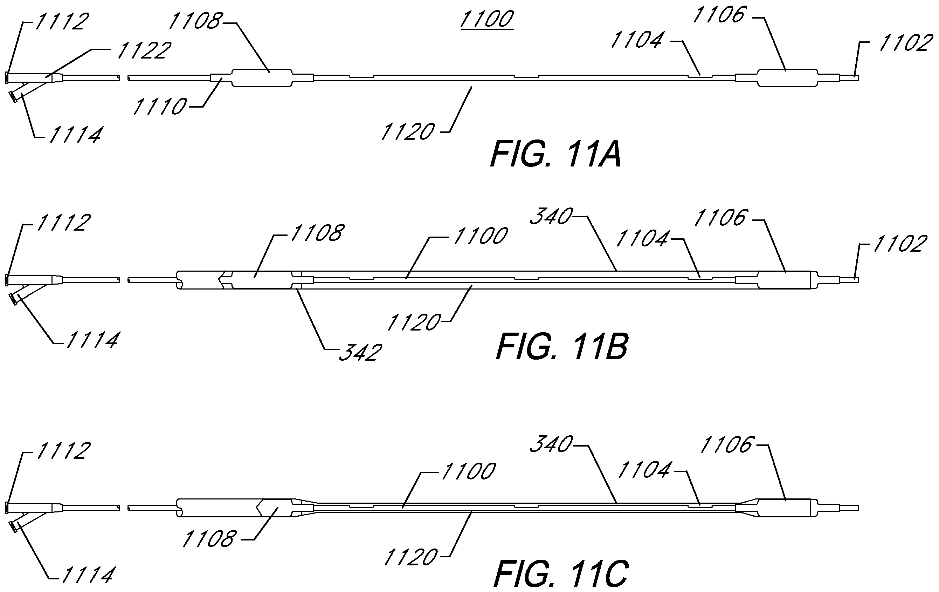

Fig. 11a illustrates a collapsing obturator 1100 for use with expandable introducer sheaths. The collapsing obturator 1100 comprises a length of obturator tubing 1102, a hub 1122 further comprising an evacuation port 1112, and a sealing balloon inflation port 1114, a proximal sealing balloon 1108 having a plurality of balloon bonds 1110, a distal sealing balloon 1106 comprising a plurality of balloon bonds 1110, a plurality of evacuation vents 1104 and an inter-balloon evacuation region 1120.

Referring to Fig. 11a, the sealing balloons 1106 and 1108 can be elastomeric balloons fabricated from materials such as, but not limited to, polyurethane, latex, silicone elastomer, thermoplastic elastomer, and the like, or they can be substantially inelastic balloons such as those fabricated from materials such as, but not limited to, polyolefin, irradiated polyethylene, polyester (PET), polyimide, polyamide, and the like. The proximal and distal sealing balloons 1108, 1106, respectively, can further be coated with conformable materials to improve sealing between the inflated balloons 1108, 1106, and the inside wall of an inflated sheath tube. Such coating (not shown) can include the same materials used to fabricate the elastomeric balloons described herein. The coating can further comprise hydrogel, or other gel-type substance.

The obturator tubing 1102 can comprise a multi-lumen cross-section or it can comprise an annular configuration having an inner tube and an outer tube with an annular lumen therebetween to operably transmit pressurized fluid to the interiors of the balloons 1106, 1108 as well as evacuating the inter-balloon region 1120 through the one or more vents 1104. The balloon pressurization port 1114 on the hub 1122 can be operably connected to a lumen and thereby to the interior of the sealing balloons 1106, 1108 by a pressurization vent or skive in the tubing wall 1102 under the region of the balloons 1106, 1108. The evacuation port 1112 can be operably connected to another, separate lumen within the tubing 1102, which is further operably connected to the one or more vent ports 1104 skived or cut into the tubing 1102 to operably connect the evacuation lumen to the outside environment.

Fig. 11B illustrates the collapsing obturator 1100 having been inserted into a diametrically expanded introducer sheath, further comprising the transition zone tubing 342, and the distal sheath tubing 340, and then pressurized to expand the two sealing balloons 1108, 1106. The proximal sealing balloon 1108 preferably resides within the proximal non-expandable region of a sheath while the distal sealing balloon 1106 preferably resides as close as possible to the distal end of the sheath so as to provide some seal but permit the maximum amount of sheath collapse proximal thereto. The inter-balloon evacuation region 1120 now defines a sealed volume with its outer boundary being the inside surface of the expanded sheath distal tubing 340 and transition zone 342.

Fig. 11C illustrates the collapsing obturator 1100 within the introducer sheath with the two sealing balloons 1106, 1108 inflated and the region between the sealing balloons 1120 but outside the collapsing obturator 1100 depressurized to radially collapse the distal, expandable introducer sheath tubing 340. Following such deflation, the sealing balloons 1106, 1108 can be deflated and the system removed from a patient with less friction and potential for tissue trauma than a sheath that is removed, fully expanded, or never collapsed. Note that a portion of the distal most region of the sheath tubing 340 remains expanded where the expanded sealing balloon 1106 was located during collapse. This short length of expanded sheath tubing 340 is easier and less traumatic to remove than a longer length of expanded sheath tubing 340. At the proximal end, the sealing balloon 1108 resides within the transition zone 342 or the proximal non-collapsible sheath tubing 338, which is outside the highly tortuous vasculature of the patient and so this has no effect on sheath removal. The distal sealing balloon 1106 can function with a minimum of about 2.5 mm (0.100 inches) of seal. A partial vacuum is drawn in the evacuation region 1120, by way of the evacuation port 1112, to collapse the outer sheath tubing 340.

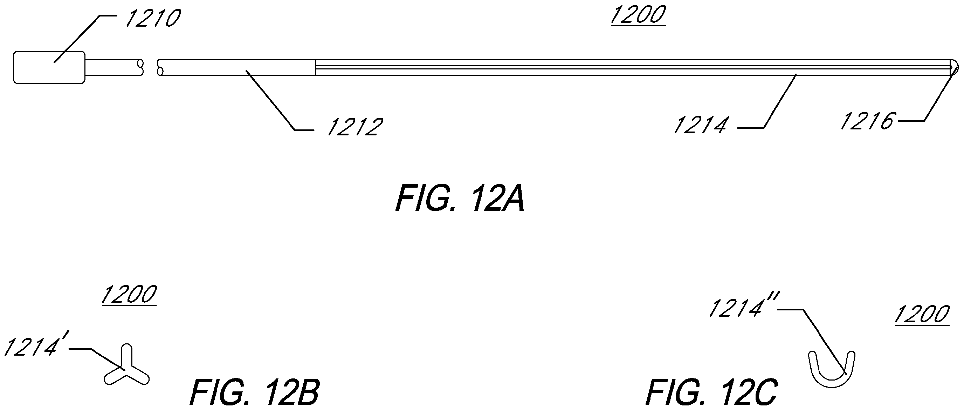

Fig. 12A illustrates a refolding or forming obturator 1200 in side view configured to control the shape of the distal collapsible region 340 of a sheath 302. The refolding obturator 1200 is configured to be re-inserted into a sheath 302 prior to re-collapse such that the re-collapse under pressure is substantially controlled. The forming obturator 1200 comprises a handle 1210, a proximal portion 1212 having a substantially round cross-section, a distal forming region 1214, and a nose cone 1216. The round proximal portion 1212 is configured to beneficially seal within a hemostasis valve within, or connected to, a sheath hub 332. The handle 1210 is configured for manual grasping by the operator. The forming obturator 1200 is preferably fabricated from flexible materials that can bend within the sheath 302 but yet retain some shape to help form the sheath distal region 340, upon re-collapse. The forming obturator 1200 can be a single integral structure or the components can be affixed to one another. The forming obturator 1200 can be fabricated from materials such as, but not limited to, stainless steel, polyethylene, polypropylene, silicone elastomer, thermoplastic elastomer, polyurethane, polyacetal, and the like. The refolding obturator 1200 can be solid or hollow, at least in part, in construction. The forming obturator 1200, in the forming region 1214 can comprise various cross-sectional shapes such as, but not limited to, a cross (as illustrated), a three-blade propeller, a U, a W, a V, and the like. The forming obturator 1200 is configured to be removable and reinserted into a sheath 302 prior to re-collapse. The forming obturator 1200 can further comprise a guidewire lumen (not shown) having a diameter of about 0.5 mm to 1.5 mm (0.020 to 0.060 inches). The forming obturator 1200 can also be termed a collapsing or refolding obturator. The forming obturator 1200 can help prevent the formation of large, stiff wings in the distal collapsible region 304 following re-collapse.

Fig. 12B illustrates a cross-sectional view of another embodiment of the forming region 1214' of a forming or refolding obturator 1200 having a three-pronged profile.

Fig. 12C illustrates a cross-sectional view of another embodiment of the forming region 1214' of a forming or collapsing obturator 1200" having a splayed U configuration.

The vascular access sheath disclosed herein has benefit in reaching far into the cerebrovasculature. Sheaths of similar construction and similar sizes are also useful for peripheral artery catheterization such as access to the popliteal arteries of the leg, arteries of the arm, or vessels leading to or from body organs. They are also useful for access to the vasculature of the heart such as the coronary arteries. Such sheaths can also be modified for use in radial artery access. The radial artery of the arm is useful for cardiac or cerebrovascular access because it shortens the distance needed to travel by the catheter and improves the approach in certain cases. The radial artery acceses sheath can beneficially include a much longer expandable (and collapsible) region reaching substantially all the way from the distal end to the proximal end of the sheath in some embodiments. Radial artery, coronary artery, and peripheral sheaths can be configured with collapsed outer profiles of about 1 mm (3 French) to about 2 mm (6 French) and can further be configured to expand to an outside diameter of about 2 mm (6 French) to about 4.3 mm (13 French), depending on the application. Since the radial artery is small in diameter, the collapsibility of the sheath is beneficial in removal of the sheath which might be otherwise bound within the artery following expansion to its full working diameter.

It also should be noted that certain objects and advantages of the invention have been described above for the purpose of disclosing the invention and the advantages achieved over the prior art. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

Moreover, although this invention has been disclosed in the context of certain preferred embodiments and examples, it will be understood by those skilled in the art that the present invention extends beyond the specifically disclosed embodiments to other alternative embodiments and/or uses of the invention. In addition, while a number of variations of the invention have been shown and described in detail, other modifications, which are within the scope of this invention, will be readily apparent to those of skill in the art based upon this disclosure. For example, it is contemplated that various combination or subcombinations of the specific features and aspects of the embodiments may be made and still fall within the scope of the invention. Accordingly, it should be understood that various features and aspects of the disclosed embodiments can be combined with or substituted for one another in order to form varying modes of the disclosed invention. Thus, it is intended that the scope of the present invention herein disclosed should not be limited by the particular disclosed embodiments described above, but should be determined only by a fair reading of the claims that follow

## Claims

1. A guide catheter for providing access to a patient's cerebrovasculature through an aortic passageway leading from an iliac or femoral location comprising:
an axially elongate sheath tube (302) having a distal portion, a proximal portion and a lumen extending therebetween, the proximal portion adapted to extend out of the patient and the distal portion adapted to extend at least to the patient's carotid arteries; the sheath tube (302) comprising at least one diametrically expandable region (340) that comprises a malleable reinforcement structure configured to maintain the at least one diametrically expandable region (340) in a first cross-sectional configuration in which the at least one diametrically expandable region (340) is longitudinally folded into a reduced cross-sectional profile and can be expanded to a second cross-sectional configuration in which the at least one diametrically expanded region (340) is unfolded into a larger cross-sectional profile;
a hub (332) coupled to the sheath tube (302) at its proximal end; and
a dilator (304) pre-inserted through the lumen in the axially elongate sheath tube (302), wherein the dilator (304) comprises a length of dilator tubing, a dilator hub (322) comprising a balloon inflation port (326) and a guidewire access port further comprising a hemostasis valve (324), and a non-compliant balloon (314), which is deflated and folded about the dilator tubing to form a minimum profile;
wherein the dilator (304) is operable to expand the at least one diametrically expandable region (340) from the first, cross-sectional configuration to the second, larger, cross-sectional configuration;
wherein the malleable reinforcement structure controls the structural configuration of the diametrically expandable region (340) prior, and subsequent, to expansion, the guide catheter **characterized by**:
an outer, substantially non-distensible layer (1010) separated from inner layers (1008) of the axially elongate tube except at locations proximal to the proximal end of the expandable region (340) and proximate the distal end of the expandable region (340), wherein pressurization of the gap separating the inner layers (1008) from the outer layer (1010) causes inward deformation, collapse, or cross-sectional area reduction of the expandable region (340), following expansion.

2. The guide catheter of Claim 1, wherein in the first cross-sectional configuration, the at least one diametrically expandable region (340) is longitudinally more flexible than in the second cross-sectional configuration.

3. The guide catheter of any one of the preceding Claims, further comprising a braided reinforcement member within a proximal end of the sheath tube (302) to provide for kink resistance and torqueability.

4. The guide catheter of any one of the preceding Claims, wherein the expandable region (340) comprises an elastic or semi-elastic wall.

5. The guide catheter of any one of the preceding Claims, wherein the expandable region (340) comprises an elastic or semi-elastic wall further reinforced with an internal braid.

6. The guide catheter of any one of the preceding Claims, further comprising hemostasis valves (352) affixed to the hub to prevent excessive loss of blood from the patient.

7. The guide catheter of any one of the preceding Claims, wherein the balloon dilator (314) comprises a non-distensible high-pressure balloon disposed along at least the entire length of the expandable region (340).

8. The guide catheter of any one of the preceding Claims, wherein the expandable guide catheter length is sufficient to span the distance from an insertion point in a femoral artery to a region within the circle of Willis, or beyond.

9. The guide catheter of any one of the preceding Claims, wherein the expandable region (340) expands from a first, smaller outside diameter of approximately 1.3 mm or less to a second, larger outside diameter of approximately 1.7 mm, or larger.

10. The guide catheter of any one of the preceding Claims, further comprising nitinol reinforcing elements within the expandable region (340).

11. The guide catheter of Claim 10, wherein the nitinol reinforcing elements are biased to diametrically expand the expandable region (340).

12. The guide catheter of Claim 10 or 11, wherein the nitinol reinforcing elements are biased to diametrically collapse the expandable region (340).

13. The guide catheter of any one of Claims 10 to 12, wherein the nitinol elements comprise shape-memory properties that are fully activated at about body temperature to expand the expandable region (340).

14. The guide catheter of any one of Claims 10 to 12, wherein the nitinol elements comprise shape-memory elements that are fully activated at temperatures above body temperature to expand the expandable region (340) or collapse the expandable region (340).

15. The guide catheter of any one of the preceding Claims, wherein the malleable reinforcement structure comprises a flat wire wound into a coil.

16. The guide catheter of any one of the preceding Claims, wherein the proximal portion comprises a coil reinforcement fabricated from spring hardness stainless steel.

17. The guide catheter of any one of the preceding Claims, wherein the proximal portion comprises both a coil and a braided reinforcement embedded within a polymeric surround.

18. The guide catheter of any one of the preceding Claims, further comprising a purge port (358) affixed to the proximal end of the sheath tube (302) and operably connected to the lumen of the guide catheter, wherein the purge port (358) comprises a valve to prevent the loss of fluid or ingress of air from the lumen when the valve is closed.

19. The guide catheter of any one of the preceding Claims, wherein the diametrically expandable region (340) comprises a malleable reinforcement structure embedded between two layers of polymer, further wherein the malleable reinforcement structure does not substantially move relative to the two layers of polymer when the sheath tube (302) is expanded from its first cross-sectional configuration to its second cross-sectional configuration.

20. The guide catheter of any one of the preceding Claims, further comprising a double outer jacket (1010) layer disposed over the inner layers (1008) of the sheath in the expandable region (340), wherein the double layer is separated by an interior region but is bonded and sealed together at locations proximal to the proximal end of the expandable region (340) and distal to the distal end of the expandable region (340); further wherein pressurization of the interior region between the double layer causes diametric collapse of the innermost of the double layer, which causes collapse of the sheath inner layers (1008) in the expandable region (340).

## Patentansprüche

1. Führungskatheter zum Bereitstellen eines Zugangs zur Zerebrovaskulatur eines Patienten durch einen von einer iliakalen oder femoralen Position wegführenden aortischen Durchgang, umfassend:
einen axial länglichen Schleusenschlauch (302) mit einem distalen Abschnitt, einem proximalen Abschnitt und einem sich dazwischen erstreckenden Lumen, wobei der proximale Abschnitt angepasst ist, um sich aus dem Patienten zu erstrecken, und der distale Abschnitt angepasst ist, um sich mindestens in die Karotisartieren des Patienten zu erstrecken;
wobei der Schleusenschlauch (302) mindestens eine diametrisch dehnbare Region (340) umfasst, die eine verformbare Verstärkungsstruktur umfasst, die konfiguriert ist, um die mindestens eine diametrisch dehnbare Region (340) in einer ersten Querschnittkonfiguration zu halten, in der die mindestens eine diametrisch dehnbare Region (340) in Längsrichtung in ein verringertes Querschnittprofil gefaltet ist und in eine zweite Querschnittkonfiguration gedehnt werden kann, in der die mindestens eine diametrisch gedehnte Region (340) in ein größeres Querschnittprofil entfaltet ist;
Ansatz (332), der am Schleusenschlauch (302) an sein proximales Ende gekoppelt ist; und
Dilatator (304) der durch das Lumen in den axial länglichen Schleusenschlauch (302) voreingeführt ist, wobei der Dilatator (304) eine Länge einer Dilatatorleitung, einen Dilatatoransatz (322), der einen Balloninflationsanschluss (326) und einen Führungsdrahtzugangsanschluss umfasst, ferner umfassend ein Hämostaseventil (324) und einen nicht-konformen Ballon (314), der entleert und um die Dilatatorleitung gefaltet ist, um ein Minimalprofil zu bilden, umfasst;
wobei der Dilatator (304) betrieben werden kann, um die mindestens eine diametrisch dehnbare Region (340) von der ersten Querschnittkonfiguration zur zweiten, größeren Querschnittkonfiguration zu dehnen;
wobei die verformbare Verstärkungsstruktur die strukturelle Konfiguration der diametrisch dehnbaren Region (340) vor und nach der Dehnung steuert, wobei der Führungskatheter **gekennzeichnet ist durch**:
eine äußere, im Wesentlichen nicht ausdehnbaren Schicht (1010), die von Innenschichten (1008) des axial länglichen Schlauchs getrennt ist, außer an Positionen proximal zum proximalen Ende der dehnbaren Region (340) und nahe dem distalen Ende der dehnbaren Region (340), wobei eine Druckbeaufschlagung des Spalts, der die Innenschichten (1008) von der Außenschicht (1010) trennt, eine nach innen gerichtete Verformung oder Verringerung der Querschnittfläche der dehnbaren Region (340) nach der Expansion bewirkt.

2. Führungskatheter nach Anspruch 1, wobei in der ersten Querschnittkonfiguration die mindestens eine diametrisch dehnbare Region (340) in Längsrichtung flexibler ist als die zweite Querschnittkonfiguration.

3. Führungskatheter nach einem der vorstehenden Ansprüche, ferner umfassend ein Geflechtverstärkungselement in einem proximalen Ende des Schleusenschlauchs (302), um für Knickresistenz und Drehstabilität zu sorgen.

4. Führungskatheter nach einem der vorstehenden Ansprüche, wobei die dehnbare Region (340) eine elastische oder halbelastische Wand umfasst.

5. Führungskatheter nach einem der vorstehenden Ansprüche, wobei die dehnbare Region (340) eine elastische oder halbelastische Wand umfasst, die ferner mit einem Innengeflecht verstärkt ist.

6. Führungskatheter nach einem der vorstehenden Ansprüche, ferner umfassend Hämostaseventile (352), die an dem Ansatz befestigt sind, um einen übermäßigen Blutverlust aus dem Patienten zu verhindern.

7. Führungskatheter nach einem der vorstehenden Ansprüche, wobei der Ballondilatator (314) einen nicht-ausdehnbaren Hochdruckballon umfasst, der entlang mindestens der gesamten Länge der dehnbaren Region (340) angeordnet ist.

8. Führungskatheter nach einem der vorstehenden Ansprüche, wobei die Länge des dehnbaren Führungskatheters ausreichend ist, um die Entfernung von einer Einführungsstelle in einer femoralen Arterie zu einer Region innerhalb des Willis-Kreises, oder darüber hinaus, zu überbrücken.

9. Führungskatheter nach einem der vorstehenden Ansprüche, wobei sich die dehnbare Region (340) von einem ersten, kleineren Außendurchmesser von ungefähr 1,3 mm oder weniger zu einem zweiten, größeren Außendurchmesser von ungefähr 1,7 mm oder größer dehnt.

10. Führungskatheter nach einem der vorstehenden Ansprüche, ferner umfassend Nitinol-Verstärkungselemente in der dehnbaren Region (340).

11. Führungskatheter nach Anspruch 10, wobei die Nitinol-Verstärkungselemente vorgespannt sind, um die dehnbare Region (340) diametrisch zu dehnen.

12. Führungskatheter nach Anspruch 10 oder 11, wobei die Nitinol-Verstärkungselemente vorgespannt sind, um die dehnbare Region (340) diametrisch zu kollabieren.

13. Führungskatheter nach einem der Ansprüche 10 bis 12, wobei die Nitinol-Elemente Formgedächtniseigenschaften umfassen, die bei etwa Körpertemperatur vollständig aktiviert sind, um die dehnbare Region (340) zu dehnen.

14. Führungskatheter nach einem der Ansprüche 10 bis 12, wobei die Nitinol-Elemente Formgedächtniselemente umfassen, die bei Temperaturen über der Körpertemperatur vollständig aktiviert sind, um die dehnbare Region (340) zu dehnen oder die dehnbare Region (340) zu kollabieren.

15. Führungskatheter nach einem der vorstehenden Ansprüche, wobei die verformbare Verstärkungsstruktur einen zu einer Spule gewickelten flachen Draht umfasst.

16. Führungskatheter nach einem der vorstehenden Ansprüche, wobei der proximale Abschnitt eine aus Federhärten-Edelstahl hergestellte Spulenverstärkung umfasst.

17. Führungskatheter nach einem der vorstehenden Ansprüche, wobei der proximale Abschnitt sowohl eine Spulen- als auch eine Geflechtverstärkung umfasst, die in einer Polymerumgebung eingebettet sind.

18. Führungskatheter nach einem der vorstehenden Ansprüche, ferner umfassend einen Spülanschluss (358), der am proximalen Ende des Schleusenschlauchs (302) befestigt und operativ mit dem Lumen des Führungskatheters verbunden ist, wobei der Spülanschluss (358) ein Ventil umfasst, um den Verlust eines Fluids oder das Eindringen von Luft aus dem Lumen zu verhindern, wenn das Ventil geschlossen ist.

19. Führungskatheter nach einem der vorstehenden Ansprüche, wobei die diametrisch dehnbare Region (340) eine verformbare Verstärkungsstruktur umfasst, die zwischen zwei Polymerschichten eingebettet ist, ferner wobei sich die verformbare Verstärkungsstruktur im Wesentlichen nicht relativ zu den beiden Polymerschichten bewegt, wenn der Schleusenschlauch (302) von seiner ersten Querschnittkonfiguration zu seiner zweiten Querschnittkonfiguration gedehnt wird.

20. Führungskatheter nach einem der vorstehenden Ansprüche, ferner umfassend eine doppelte Außenhüllenschicht (1010), die über den Innenschichten (1008) der Schleuse in der dehnbaren Region (340) angeordnet ist, wobei die doppelte Schicht durch eine innere Region getrennt, aber an Positionen proximal zum proximalen Ende der dehnbaren Region (340) und distal zum distalen Ende der dehnbaren Region (340) verbunden und gedichtet ist;
ferner wobei eine Druckbeaufschlagung der inneren Region zwischen der doppelten Schicht ein diametrisches Kollabieren des Innersten der doppelten Schicht bewirkt, was ein Kollabieren der Hülseninnenschichten (1008) in der dehnbaren Region (340) bewirkt.

## Revendications

1. Cathéter de guidage permettant d'accéder au système cérébrovasculaire d'un patient par l'intermédiaire d'un passage aortique partant d'un emplacement iliaque ou fémoral comprenant :
un tube de gaine axialement allongé (302) possédant une partie distale, une partie proximale et une lumière s'étendant entre celles-ci, la partie proximale étant conçue pour s'étendre hors du patient et la partie distale étant conçue pour s'étendre au moins jusqu'aux artères carotides du patient ; le tube de gaine (302) comprenant au moins une zone diamétralement extensible (340) qui comprend une structure de renforcement malléable conçue pour maintenir ladite au moins une zone diamétralement extensible (340) dans une première configuration transversale dans laquelle ladite au moins une zone diamétralement extensible (340) est pliée longitudinalement en une profil transversal réduit et peut être déployée dans une seconde configuration transversale dans laquelle ladite au moins une zone diamétralement extensible (340) est dépliée en un profil transversal plus grand ;
un moyeu (332) couplé au tube de gaine (302) au niveau de son extrémité proximale ; et
un dilatateur (304) pré-inséré à travers la lumière dans le tube de gaine axialement allongé (302), ledit dilatateur (304) comprenant une longueur de tube de dilatateur, un moyeu de dilatateur (322) comprenant un orifice de gonflage de ballonnet (326) et un orifice d'accès de fil-guide comprenant en outre une valve hémostatique (324), et un ballonnet non flexible (314), qui est dégonflé et plié autour du tube de dilatateur pour former un profil minimal ;
ledit dilatateur (304) étant actionnable pour étendre ladite au moins une zone diamétralement extensible (340) de la première configuration transversale à la seconde configuration transversale plus grande ;
ladite structure de renforcement malléable contrôlant la configuration structurelle de la zone diamétralement extensible (340) avant, et après, le déploiement, le cathéter de guidage étant **caractérisé par** :
une couche externe pratiquement non extensible (1010) distincte des couches internes (1008) du tube axialement allongé sauf au niveau des emplacements proximaux par rapport à l'extrémité proximale de la zone extensible (340) et à proximité de l'extrémité distale de la zone extensible (340), la pressurisation de l'espace séparant les couches internes (1008) de la couche externe (1010) provoquant une déformation vers l'intérieur, un affaissement, ou une réduction de surface transversale de la zone extensible (340), suite au déploiement.

2. Cathéter de guidage selon la revendication 1, dans la première configuration transversale, ladite au moins une zone diamétralement extensible (340) étant longitudinalement plus flexible que dans la seconde configuration transversale.

3. Cathéter de guidage selon l'une quelconque des revendications précédentes, comprenant en outre un élément de renforcement tressé au sein d'une extrémité proximale du tube de gaine (302) pour assurer la résistance au pliage et l'aptitude à la torsion.

4. Cathéter de guidage selon l'une quelconque des revendications précédentes, ladite zone extensible (340) comprenant une paroi élastique ou semi-élastique.

5. Cathéter de guidage selon l'une quelconque des revendications précédentes, ladite zone extensible (340) comprenant une paroi élastique ou semi-élastique renforcée en outre par un tressage interne.

6. Cathéter de guidage selon l'une quelconque des revendications précédentes, comprenant en outre des vannes hémostatiques (352) fixées au moyeu pour empêcher une perte de sang excessive du patient.

7. Cathéter de guidage selon l'une quelconque des revendications précédentes, ledit dilatateur de ballonnet (314) comprenant un ballonnet haute pression non extensible disposé le long d'au moins la longueur entière de la zone extensible (340).

8. Cathéter de guidage selon l'une quelconque des revendications précédentes, ladite longueur de cathéter de guidage extensible étant suffisante pour couvrir la distance d'un point d'insertion dans une artère fémorale jusqu'à une zone située au sein du cercle de Willis, ou au-delà.

9. Cathéter de guidage selon l'une quelconque des revendications précédentes, ladite zone extensible (340) s'étendant d'un premier diamètre externe plus petit inférieur ou égal à environ 1,3 mm à un second diamètre externe plus grand supérieur ou égal à environ 1,7 mm.

10. Cathéter de guidage selon l'une quelconque des revendications précédentes, comprenant en outre des éléments de renforcement en nitinol au sein de la zone extensible (340).

11. Cathéter de guidage selon la revendication 10, lesdits éléments de renforcement en nitinol étant inclinés pour étendre diamétralement la zone extensible (340).

12. Cathéter de guidage selon la revendication 10 ou 11, lesdits éléments de renforcement en nitinol étant inclinés pour affaisser diamétralement la zone extensible (340).

13. Cathéter de guidage selon l'une quelconque des revendications 10 à 12, lesdits éléments en nitinol comprenant des propriétés de mémoire de forme qui sont complètement activées à une température à peu près corporelle pour étendre la zone extensible (340).

14. Cathéter de guidage selon l'une quelconque des revendications 10 à 12, lesdits éléments en nitinol comprenant des éléments à mémoire de forme qui sont complètement activés à des températures supérieures à la température corporelle pour étendre la zone extensible (340) ou affaisser la zone extensible (340).

15. Cathéter de guidage selon l'une quelconque des revendications précédentes, ladite structure de renforcement malléable comprenant un enroulement de fil plat au sein d'une bobine.

16. Cathéter de guidage selon l'une quelconque des revendications précédentes, ladite partie proximale comprenant un renforcement de bobine fabriqué à partir d'acier inoxydable à dureté de ressort.

17. Cathéter de guidage selon l'une quelconque des revendications précédentes, ladite partie proximale comprenant à la fois une bobine et un renforcement tressé incorporé au sein d'un environnement polymère.

18. Cathéter de guidage selon l'une quelconque des revendications précédentes, comprenant en outre un orifice de purge (358) fixé à l'extrémité proximale du tube de gaine (302) et relié fonctionnement à la lumière du cathéter de guidage, ledit orifice de purge (358) comprenant une valve pour empêcher la perte de fluide ou la pénétration d'air à partir de la lumière lorsque la valve est fermée.

19. Cathéter de guidage selon l'une quelconque des revendications précédentes, ladite zone diamétralement extensible (340) comprenant une structure de renforcement malléable incorporée entre deux couches de polymère, en outre ladite structure de renforcement malléable ne se déplaçant pratiquement pas par rapport aux deux couches de polymère lorsque le tube de gaine (302) est déployé de sa première configuration transversale à sa seconde configuration transversale.

20. Cathéter de guidage selon l'une quelconque des revendications précédentes, comprenant en outre une double couche d'enveloppe externe (1010) disposée sur les couches internes (1008) de la gaine dans la zone extensible (340), ladite double couche étant séparée par une zone interne mais étant collées et scellées ensemble au niveau d'emplacements proximaux par rapport à l'extrémité proximale de la zone extensible (340) et distaux par rapport à l'extrémité distale de la zone extensible (340) ; en outre ladite pressurisation de la zone interne entre la double couche provoquant l'affaissement diamétral de l'intérieur de la double couche, qui provoque l'affaissement des couches internes de gaine (1008) dans la zone extensible (340).
